# EUROPEAN PATENT APPLICATION

(11) **EP 4 194 008 A1**
(43) Date of publication of application: **14.06.2023**
(21) Application number: 21852796.8
(22) Date of filing: 04.08.2021
(51) Int. Cl.: A61K 39/39, A61K 39/00

(54) **KINETICALLY ACTING ADJUVANT ENSEMBLE**

(30) Priority: 04.08.2020 KR 20200097551; 03.08.2021 KR 20210102216
(71) Applicant: PROGENEER INC., Guro-gu Seoul 08380 (KR)
(72) Inventor: LIM, Yong Taik, Seongnam-si, Gyeonggi-do 13597 (KR); JIN, Seung Mo, Seoul 06709 (KR); YOO, Yeon Jeong, Seongnam-si, Gyeonggi-do 13559 (KR)
(74) Representative: Dehns
(86) International application number: PCT/KR2021/010239
(87) International publication number: WO 2022/031011

(57) **Abstract**

The present invention relates to an adjuvant ensemble in which two or more immunostimulatory substances for inducing the activation of various immune cells are combined, and to a novel concept of a kinetically acting adjuvant ensemble and use thereof, the treatment order and the time interval are adjusted to maximize the finally exhibited synergistic immunostimulatory response. The kinetically acting is characterized in that the second immunostimulatory substance responds to intrinsic factors (enzymes, redox potential, GSH, and pH) and extrinsic factors (redox, pH, temperature, photo/light, magnetism, ultrasound, and electrical response) in immune cells, and thus chemical binding at a binding site is cleaved, and the active site is exposed so that function is kinetically recovered, or two or more immunostimulatory substances are sequentially released from a carrier at regular time intervals, thus inducing immune activity. In such a novel immunostimulatory adjuvant ensemble, a first immunostimulatory substance and a second immunostimulatory substance can be selected in a personalized manner according to applications, and the adjuvant ensemble can be used for anticancer effects and the development of drugs for the prevention and treatment of infectious diseases.

## Description

### [Technical Field]

The present invention relates to a novel adjuvant ensemble including two or more immune activating materials that activate immune cells and whose action time is kinetically controlled, and more particularly, to an adjuvant ensemble designed to first induce an immune response by binding of a first immune activating material to a receptor, and sequentially induce an immune response by binding of a second immune activating material to a receptor.

### [Background Art]

Immune response is a series of responses of activated immune cells against exogenous and endogenous materials, that is, antigens, and when microorganisms such as bacteria or viruses, or foreign bio-substances are introduced into the body, they are recognized by immune cells, which are then activated to secrete a factor such as a cytokine, thereby causing an inflammatory response. Recently, active research on mechanisms in an innate immune response stage that non-specifically acts at the early stage of infection is actively progressing, and among these mechanisms, a toll-like receptor (TLR), which is a gene capable of recognizing a pathogen in the early stage of inflammation, is known to recognize a cell membrane component of a pathogen or a nucleic acid component to induce an immune response, and by using the TLR, studies on various toll-like receptor ligands (TLR ligands) for activating immune cells are actively progressing (US Patent Publication No. 2012-0294885).

Toll-like receptor agonists are known to effectively induce not only humoral immunity but also cellular immunity as toll-like receptor agonists in endosomes. However, it is difficult to disperse such multifunctional toll-like receptor agonists in an aqueous solution due to their molecular structure. In addition, since they are dissolved only in specific organic solvents such as DMSO and methanol, and not dissolved in generally used organic solvents, there are limitations in manufacturing immune-activating drugs in various formulations. Therefore, a cream-type formulation (e.g., Aldara cream) in which various surfactants were mixed has been commercialized. In some studies, to overcome the above problems, such agonists are prepared in the form of a salt and thus can be dissolved in an aqueous solution. However, toll-like receptor agonists prepared in the form of a salt are absorbed into the blood vessel in the body to induce an immune response in the blood vessel (systemic immune response), thereby causing many side effects (e.g., cytokine storm, various non-specific hypersensitive immune responses, etc.), so it is not easy to use. In addition, due to such side effects, to be actually used in treatment, it has to be treated at a smaller concentration than the effective dose, so it also becomes a factor in decreasing efficacy. To overcome such problems, some pharmaceutical companies are trying to prevent directly absorption into blood vessels by introducing a lipophilic lipid, or by direct chemical bonding to a polymer chain having a large size. However, since toll-like receptor agonists prepared by such a method have their active site still exposed to the outside, they induce non-specific immune responses in the body and still have the possibility of inducing toxicity.

When various materials for inducing innate immunity, including such toll-like receptor agonists, are mixed and used, the increase in effect of an innate immune response continues to be reported. In particular, compared to the case in which toll-like receptor ligands having different signaling systems are independently used in an immune activation mechanism, when a combination of two or more toll-like receptor ligands is used, it has been reported that the capacity of activating immune cells increases 20 to 50-fold or more. However, since innate immunity-inducing materials that have different signaling systems have different mechanisms, depending on the order of treatment and/or treatment time interval, the effect of an immune activation response appears very differently. However, since separate administration of a plurality of innate immunity-inducing materials, that is, adjuvants, into the body at predetermined time intervals is not only impractical, but has very low probability that multiple separately-administered adjuvants stimulate the same immune cell to increase an immune activation effect, in practice, it is not easy to administer different types of innate immunity-inducing materials at different times (asynchronous treatment) in the medical field.

Accordingly, since the time interval for immune activation may be optimized by binding of each adjuvant injected into the body to each receptor at predetermined time intervals while synchronously treating multiple adjuvants, the development of an adjuvant ensemble that can optimize an immune activation effect can lead to a remarkably improved vaccine effect, and thus it is expected to have a great ripple effect in the next-generation vaccine market.

### [Disclosure]

### [Technical Problem]

To solve the problems in the related art, the present invention is directed to providing a kinetically acting adjuvant ensemble designed so that different adjuvants, for example, a toll-like receptor agonist, saponin, an anti-viral peptide, an inflammasome inducer, a NOD ligand, a cytosolic DNA sensor (CDS) ligand, and a stimulator of interferon genes (STING) ligand, act in a predetermined order and at predetermined time intervals, and a use thereof.

However, technical problems to be solved in the present invention are not limited to the above-described problems, and other problems which are not described herein will be fully understood by those of ordinary skill in the art from the following descriptions.

### [Technical Solution]

The present invention provides a kinetically acting adjuvant ensemble composition, which includes two or more types of adjuvants, wherein, a first adjuvant first binds to an immune cell receptor to induce a first immune response, and a second adjuvant that is a complex in which a cleavable linker binds to the active site, sequentially binds to an immune cell receptor to induce a second immune response.

In one embodiment of the present invention, the kinetic control is characterized in that when a cleavable linker binds to the active site of a second adjuvant to maintain an inactive state, and then the cleavable linker blocking the active site is cleaved within preferably 2 to 12 hours, and more preferably 3 to 9 hours, the activity of the immune modulator is temporally delayed. The adjuvant ensemble composition of the present invention may provide a kinetic ensemble acting at predetermined time intervals when two or more types of immune activating materials were synchronously administered, thereby eliciting the maximum synergistic effect of an immunological response. The kinetic ensemble may act on a molecular scale and/or macro scale.

In another embodiment of the present invention, the cleavable linker may be comprise any one or more bonds selected from the group consisting of a disulfide, a carbamate, a hydrazine, an ester, a peptide, an azide, an amide, a hydrazone, a thioether, a phosphodiester, a thioketal, and a combination thereof. However, there is no limitation to the type of linker as long as it can be cleaved by endogenous factors (enzyme, redox potential, GSH, pH, etc.) and/or exogenous factors (redox, pH, temperature, photo/light, magnetic, ultrasound, electrical responsive, etc.) in body.

In still another embodiment of the present invention, the chemical bond at the binding site is cleaved by any one or more factors selected from the group consisting of an enzyme, a pH, a redox potential, a temperature, ultrasonic waves, a magnetic force, and a light source.

In yet another embodiment of the present invention, the cleavable linker further includes an alkyl derivative such as ethylene oxide or ethylene glycol at one or both ends thereof, thereby increasing the solubility and flexibility of a complex in an aqueous solution.

In yet another embodiment of the present invention, to an end of the cleavable linker, any one or more materials selected from the group consisting of cholesterol, a lipid, a protein, an amino acid, a peptide and an oligonucleotide is bound, and the material may be any one of various materials having a hydrophilic or lipophilic group, serving to block the active site of the second adjuvant.

In yet another embodiment of the present invention, the second adjuvant is loaded into any one or more drug delivery systems selected from the group consisting of a nanoliposome, a nanoemulsion, a nanomicelle, a hydrogel, a scaffold, a solid nanoparticle and a polymeric nanoparticle. The loading may be simply being contained within regardless of binding, inserting into a nanoparticle structure, or binding to the nanoparticle structure, but it is not limited as long as it is a form that includes the adjuvant of the present invention.

In yet another embodiment of the present invention, the drug delivery system further includes a first adjuvant.

In yet another embodiment of the present invention, the drug delivery system further includes a ligand that can react with a receptor present on the surface of immune cells, or in an endosome or cytosol.

In yet another embodiment of the present invention, the drug delivery system further includes any one or more immune activating materials selected from the group consisting of a toll-like receptor agonist, saponin, an antiviral peptide, an inflammasome inducer, a NOD ligand, a cytosolic DNA sensor (CDS ligand), a stimulator of interferon genes (STING) ligand, an outer wall component of a pathogen, alum, a lipid, a combination thereof, and a derivative thereof.

In yet another embodiment of the present invention, the second adjuvant is preferably a toll-like receptor agonist, and more preferably any one or more selected from the group consisting of a toll-like receptor 1 agonist, a toll-like receptor 2 agonist, a toll-like receptor 3 agonist, a toll-like receptor 4 agonist, a toll-like receptor 5 agonist, a toll-like receptor 6 agonist, a toll-like receptor 7 or 8 agonist, and a toll-like receptor 9 agonist.

In yet another embodiment of the present invention, the first adjuvant may be any one or more immune activating materials selected from the group consisting of a toll-like receptor agonist, saponin, an antiviral peptide, an inflammasome inducer, a NOD ligand, a CDS ligand, a STING ligand, an outer wall component of a pathogen, alum, a lipid, a combination thereof, and a derivative thereof.

In yet another embodiment of the present invention, the immune cells are any one or more selected from the group consisting of antigen-presenting cells (dendritic cells or macrophages), natural killer (NK) cells, T cells, B cells, regulatory T cells, myeloid derived suppressor cells (MDSCs), and M2 macrophages.

In yet another embodiment of the present invention, the drug delivery system may be an ensemble designed to sequentially release two or more types of adjuvants, and have a core-shell structure having a layered structure of two or more of a liposome, a micelle, an emulsion, a self-assembled particle, and a polymeric nanoparticle, including a first adjuvant, which is first released, and a second adjuvant, which is secondarily released.

In yet another embodiment of the present invention, the drug delivery system may include stimuli-responsive block, and the stimuli may include endogenous factors (enzyme, redox potential, GSH, pH, etc.) in cells, exogenous factors (redox, pH, temperature, photo/light, magnetic, ultrasound, electrical responsive, etc.), and various physiological environment/immune factors in the body. In addition, the drug delivery system may include two or more types of stimuli-responsive blocks, which may sequentially respond to different stimuli. In addition, the stimuli-responsive blocks may sequentially respond according to the intensity of a stimulus.

In addition, the present invention provides a pharmaceutical composition for preventing or treating an infectious disease, cancer, metabolic syndrome, an autoimmune disease, or a rare disease, which includes the adjuvant ensemble composition as an active ingredient.

In one embodiment of the present invention, the pharmaceutical composition may further include an antigen, a chemotherapeutic agent, or an immune checkpoint inhibitor.

In another embodiment of the present invention, the antigen may be one or more selected from the group consisting of a protein, a recombinant protein, a glycoprotein, a gene, a peptide, a polysaccharide, a lipopolysaccharide, a polynucleotide, a cell, a cell lysate, a bacterium, and a virus.

In still another embodiment of the present invention, the pharmaceutical composition inhibits cancer growth, metastasis, recurrence, or resistance to anticancer therapy.

In addition, the present invention provides a method of preventing or treating an infectious disease, cancer, metabolic syndrome, an autoimmune disease, or a rare disease, which includes administering a composition including the adjuvant ensemble composition as an active ingredient to a subject.

In addition, the present invention provides a use of a composition including the adjuvant ensemble composition as an active ingredient for preventing or treating an infectious disease, cancer, metabolic syndrome, an autoimmune disease, or a rare disease.

In addition, the present invention provides a use of the adjuvant ensemble composition for manufacturing a medicament used in prevention or treatment of an infectious disease, cancer, metabolic syndrome, an autoimmune disease, or a rare disease.

### [Advantageous Effects]

In the present invention, compared to conventional treatment, which is synchronous administration of two materials, a novel adjuvant whose acting time is kinetically controlled can not only improve a synergistic effect, but minimize a potential toxicity problem of an adjuvant. Particularly, an ensemble in which two or more types of customized and selected immune activating materials are combined is expected to be widely applied to anticancer vaccines and effective drugs for preventing and treating various diseases, including an infectious disease due to an improved immune enhancing effect.

### [Description of Drawings]

FIG. 1 is a conceptual diagram of an adjuvant ensemble for kinetically controlling immune function on a macro-scale.
FIGS. 2 show an example of an adjuvant (toll-like receptor 7/8 agonist) for kinetically controlling immune function on a molecular scale. The upper diagram is a conceptual diagram of a kinetically acting toll-like receptor 7/8 agonist, and the lower diagram shows the characteristics of cleavable linkers.
FIG. 3 is a schematic diagram of a mechanism in which a kinetically controllable cholesterol-toll-like receptor 7 or 8 agonist complex acts differently from a conventional immune function modulator after application to immune cells (dendritic cells).
FIG. 4 shows the result of confirming the endocytosis of a nanoliposome including a cholesterol-toll-like receptor 7 or 8 agonist complex according to one embodiment of the present invention by an IL-12 level.
FIG. 5 shows the result of confirming GILT expression in antigen-presenting cells by RT-PCR according to one embodiment of the present invention.
FIG. 6 shows the result of confirming whether the chemical bond of a nanoliposome including a cholesterol-toll-like receptor 7 or 8 agonist complex is cleaved by GILT according to one embodiment of the present invention.
FIG. 7 shows the result of confirming the control of immune cells of a nanoliposome including a cholesterol-toll-like receptor 7 or 8 agonist complex according to one embodiment of the present invention.
FIG. 8 shows the result of confirming the effect of a nanoliposome including a cholesterol-toll-like receptor 7 or 8 agonist complex on the maturation degree of dendritic cells according to one embodiment of the present invention.
FIG. 9 shows the result of confirming the continuous immune response-inducing effect of a nanoliposome including a cholesterol-toll-like receptor 7 or 8 agonist complex according to one embodiment of the present invention.
FIG. 10 shows the result of confirming the differentiation-inducing effect of a nanoliposome including a cholesterol-toll-like receptor 7 or 8 agonist complex according to one embodiment of the present invention by the Th1 response of CD4+ T cells.
FIGS. 11 show the result of confirming the effect of co-administration of a toll-like receptor 3 agonist and a toll-like receptor 7 or 8 agonist by an amount of IL-12 secretion according to one embodiment of the present invention.
FIGS. 12 show the result of confirming the effect of co-administration of a toll-like receptor 4 agonist and a toll-like receptor 7 or 8 agonist by an amount of IL-12 secretion according to one embodiment of the present invention.
FIG. 13 shows the result of confirming the immune activation efficacy of an adjuvant ensemble by amounts of TNF-α and IL-6 secretion according to one embodiment of the present invention.
FIG. 14 shows the result of confirming the maturation degree of cells mediated by an adjuvant ensemble by expression levels of co-stimulatory molecules according to one embodiment of the present invention.
FIG. 15 shows the result of confirming the continuous immune response-inducing effect by an adjuvant ensemble according to one embodiment of the present invention.
FIG. 16 shows the result of confirming the degree of stabilization of an adjuvant mixture by EMSA according to one embodiment of the present invention.
FIG. 17 shows the result of confirming the stability of an adjuvant mixture in cells according to one embodiment of the present invention.
FIG. 18 shows the result of confirming the continuous immune response inducing efficacy of an adjuvant mixture *in vivo* according to one embodiment of the present invention.
FIG. 19 shows the result of confirming the antitumor effect of an adjuvant mixture *in vivo* according to one embodiment of the present invention.
FIG. 20 shows the result of analyzing immune cells recruited to tumor tissue and a lymph node by an adjuvant mixture according to one embodiment of the present invention.
FIG. 21 shows the result of analyzing immune cells recruited to tumor tissue and a lymph node by an adjuvant mixture according to one embodiment of the present invention.
FIG. 22 shows the result of analyzing immune cells recruited to tumor tissue and a lymph node by an adjuvant mixture according to one embodiment of the present invention.
FIG. 23 shows the result of analyzing immune cells recruited to tumor tissue and a lymph node by an adjuvant mixture according to one embodiment of the present invention.
FIG. 24 shows the result of confirming the immune response-inducing effect of an adjuvant mixture in a lymph node by cytokine secretion ability according to one embodiment of the present invention.
FIG. 25 shows the result of confirming the immune response-inducing effect of an adjuvant mixture in tumor tissue by cytokine secretion ability according to one embodiment of the present invention.
FIG. 26 shows the result of confirming the effect of IL-12 on the immune-activating efficacy of an adjuvant mixture according to one embodiment of the present invention.
FIG. 27 shows the result of confirming the antitumor efficacy against metastatic cancer via local inoculation with an adjuvant mixture according to one embodiment of the present invention.
FIG. 28 shows the result of confirming the lung metastasis-inhibiting effect via local inoculation with an adjuvant mixture according to one embodiment of the present invention.
FIG. 29 shows the result of confirming the antitumor efficacy against orthotopic tumors via local inoculation with an adjuvant mixture according to one embodiment of the present invention.
FIG. 30 shows the result of confirming the antitumor efficacy of the combination of an adjuvant mixture and immune checkpoint inhibitor therapy.
FIG. 31 shows the result of confirming the antitumor efficacy of the combination of an adjuvant mixture and chemotherapy according to one embodiment of the present invention.

### [Best Modes of the Invention]

As a result of intensive research on a kinetically controllable adjuvant ensemble system that can adjust the activation time of each adjuvant to increase its effect as much as possible when immune activation is induced using a combination of adjuvants after confirming that, when two or more adjuvants are used in combination, an order and a time difference act as important factors in the synergistic effect of the adjuvants, the present inventors invented a kinetically controllable adjuvant ensemble composition, which acts in such a manner that a first adjuvant acts first and a second adjuvant has a cleavable linker binding to its active site, thereby keeping its activity temporarily inhibited, and after administered into the body and reaching desired tissue or cells, for example, within 2 to 12 hours, the linker is cleaved and thereby the activity of the adjuvant is secondarily exhibited.

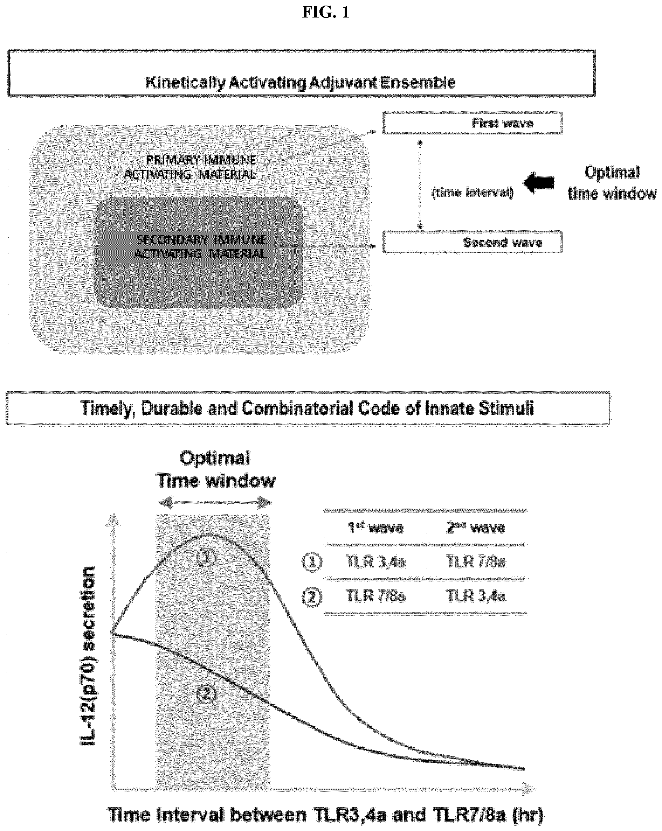

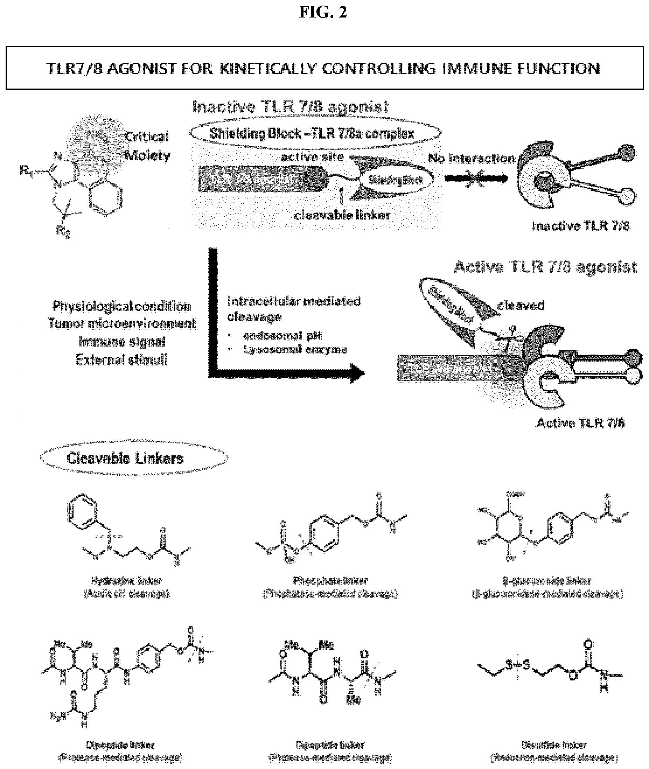

As shown in FIGS. 1 and 2, the adjuvant ensemble composition of the present invention may be a system that can control both on a molecular scale and a macro scale, in which a cleavable linker is linked to the active site of the second adjuvant, thereby keeping it inactive, and a cleavable linker blocking the active site is cleaved within 2 to 12 hours, and more preferably, 3 to 9 hours, and thus the activity of an immune activating material is temporally delayed, and the adjuvant ensemble composition of the present invention may elicit the maximum synergistic effect of an immunological response.

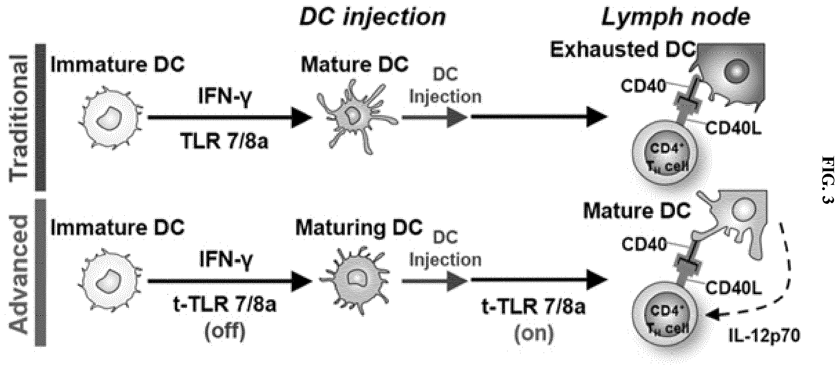

In addition, as shown in FIG. 3, the adjuvant ensemble composition of the present invention may induce a continuous immune response by maintaining dendritic cells in a state capable of inducing an immune response, that is, a maturing state for a long time, not a state incapable of inducing an immune response, that is, an exhaustion state. In addition, in the process of encountering naïve T cells in a lymph node and presenting an antigen, together with the action of CD40-CD40L, through the induction of strong immune activation, a Th1 immune response-inducing cytokine, such as IL-12, may be effectively induced.

The term "adjuvant" is the generic term for immune activating materials (immune modulator) that activate, induce, or restore normal immune function in the immune system. The adjuvant refers to a material that is used together with an antigen in order to improve immune responses, and may be used with an antigen to increase antibody production, and increase humoral and/or cellular immunity. The immune activating materials preferably include a toll-like receptor agonist, saponin, an antiviral peptide, an inflammasome inducer, a NOD ligand, a cytosolic DNA sensor (CDS ligand), a stimulator of interferon genes (STING) ligand, an emulsion, alum, Freund's incomplete adjuvant, Freund's adjuvant, or a combination thereof, and more preferably, a toll-like receptor agonist.

The "toll-like receptor agonist" used herein is a ligand directly or indirectly acting on a toll-like receptor, which is a membrane protein involved in innate immunity, and refers to a component that can cause a signaling response using a signaling pathway through the generation of an endogenous or exogenous ligand. The toll-like receptor agonist used herein may be a natural toll-like receptor agonist or a synthetic toll-like receptor agonist. The toll-like receptor agonist used herein may be a toll-like receptor 1 agonist, a toll-like receptor 2 agonist, a toll-like receptor 3 agonist, a toll-like receptor 4 agonist, a toll-like receptor 5 agonist, a toll-like receptor 6 agonist, a toll-like receptor 7 or 8 agonist, or a toll-like receptor 9 agonist.

The toll-like receptor 1 agonist refers to a ligand that can induce a signaling response by means of TLR-1, and may be, but is not limited to, a tri-acylated lipid peptide (LP); a phenol-soluble modulin; a lipid peptide of *Mycobacterium tuberculosis*; S-(2,3-bis(palmitoyloxy)-(2-RS)-propyl)-N-palmitoyl-(R)-Cys-(S)-Ser-(S)-Lys(4)-OH; a lipid peptide of *Borrelia burgdorfei*; or a trihydrochloride (Pam3Cys) lipid peptide mimicking an acetylated amino terminus of an OspA lipid peptide.

The toll-like receptor 2 agonist refers to a ligand that can induce a signaling response by means of TLR-2, and may be, but is not limited to, peptidoglycan, zymosan, HSP70, HMGB1, HA, or bam3Cys-Lip.

The toll-like receptor 3 agonist refers to a ligand that can induce a signaling response by means of TLR-3, and may be, but is not limited to, the poly(I:C) series, for example, poly(I:C), poly(ICLC), poly(IC12U), or Ampligen.

The toll-like receptor 4 agonist refers to a ligand that can induce a signaling response by means of TLR-4, and may be, but is not limited to, an outer membrane protein construct of *Shigella flexineri,* AGP, CRX-527, MPLA, PHAD, 3D-PHAD, GLA, or LPS.

The toll-like receptor 5 agonist refers to a ligand that can induce a signaling response by means of TLR-5, and may be, but is not limited to, flagellin.

The toll-like receptor 6 agonist refers to a ligand that can induce a signaling response by means of TLR-6, and may be, but is not limited to, a diacyl lipopeptide or lipoteichoic acid.

The toll-like receptor 7 or 8 agonist refers to a ligand that can induce a signaling response by means of TLR-7 or 8, and may be, but is not limited to, an imidazoquinoline-based agonist, a 8-hydroxyadenine-based agonist, a pteridone-based agonist, a 2-aminopyrimidine-based agonist, a benzoazepine-based agonist, or a 7-thia-8-oxoguanosine-based agonist, and the imidazoquinoline-based compound includes compound types disclosed in WO 2018 196823, WO 2011 049677, WO 2011 027022, WO 2017 102652, and WO 2019 040491, or pharmaceutically acceptable salts thereof, but the present invention is not limited thereto. In addition, the 8-hydroxyadenine-based compounds include compound types disclosed in WO 2012 080730, WO 2013 068438, WO 2019 036023, WO 2019 035969, WO 2019 035970, WO 2019 035971, WO 2019 035968, CN 108948016, US 2014 8846697, WO 2016 023511, WO 2017 133683, WO 2017 133686, WO 2017 133684, WO 2017 133687, WO 2017 076346, WO 2018 210298, WO 2018 095426, WO 2018 068593, WO 2018 078149, and WO 2018 041763, or pharmaceutically acceptable salts thereof, but the present invention is not limited thereto. The pteridone-based compounds include compound types disclosed in US 2010 0143301, WO 2016 007765, WO 2016 044182, WO 2017 035230, WO 2017 219931, WO 2011 057148, and CN 1087 94486, or pharmaceutically acceptable salts thereof, but the present invention is not limited thereto. The 2-aminopyrimidine-based compounds include compound types disclosed in WO 2010 133885, WO 2012066335, WO 2012 066336, WO 2012 067268, WO 2013 172479, WO 2012 136834, WO 2014 053516, WO 2014 053595, US 2018 0215720, WO 2012 156498, WO 2014 076221, WO 2016 141092, WO 2018 045144, WO 2015 014815, WO 2018 233648, WO 2014 207082, WO 2014 056593, WO 2018 002319 and WO 2013 117615, or pharmaceutically acceptable salts thereof, but the present invention is not limited thereto. The benzoazepine-based compounds include compound types disclosed in WO 2007 024612, WO 2010 014913, WO 2010 054215, WO 2011 022508, WO 2011 022509, WO 2012 097177, WO 2012 097173, WO 2016 096778, WO 2016 142250, WO 2017 202704, WO 2017 202703, WO 2017 216054, WO 2017 046112 and WO 2017 197624, or pharmaceutically acceptable salts thereof, but the present invention is not limited thereto. The 7-thia-8-oxoguanosine-based compounds include compound types disclosed in WO 2016 180691, WO 2016 055553, WO 2016 180743 and WO 2016 091698, or pharmaceutically acceptable salts thereof, but the present invention is not limited thereto. Other than the above, the toll-like receptor 7 or 8 compounds may also include toll-like receptor 7 or 8 compounds disclosed in PCT/US2009/035563, PCT/US2015/028264, PCT/US2016/020499, WO 2015 023598 and PCT/US 2015/039776, or pharmaceutically salt thereof. Alternatively, the toll-like receptor 7 or 8 agonists may include, but are not limited to, imiquimod, resiquimod, dactolisib, gardiquimod, sumanirole, motolimod, vesatolimod, loxoribine, SM360320, CL264, 3M-003, IMDQ, and Compound 54, and include all toll-like receptor 7 or 8 agonists which can be readily surmised by those of ordinary skill in the art.

The toll-like receptor 9 agonist refers to a ligand that can induce a signaling response by means of TLR-9, and may be, but is not limited to, an immune stimulating oligonucleotide. The immune stimulating oligonucleotide may include one or more CpG motifs, but the present invention is not limited thereto.

The "saponin" used herein is an amphipathic glycoside, and acts as a surfactant. For example, the saponin may be, but is not limited to, QS21, Quil A, QS7, QS17, β-escin, or digitonin.

The "antiviral peptide" used herein is the generic term for peptides exhibiting an antiviral effect, and may be, for example, KLK, but the present invention is not limited thereto.

The "inflammasome inducer" used herein is the generic term for materials that induce an inflammasome, which is a protein complex recognizing and activating a danger signal in the cytoplasm of a eukaryotic cell, and may be, for example, trehalose-6,6-dibehenate (TDB), but the present invention is not limited thereto.

The "NOD ligand" used herein is the generic term for ligands activating a Nod-like receptor, and may be, for example, M-TriLYS or N-glycosylated muramyl dipeptide, but the present invention is not limited thereto.

The "cytosolic DNA sensor (CDS) ligand" used herein is the generic term for ligands activating cGAS, which is a DNA sensor, and may be, for example, poly(dA:dT), but the present invention is not limited thereto.

The "stimulator of interferon genes (STING) ligand" used herein is the generic term for ligands activating STING, which is a sensor used by immune cells to detect cancer, and may be, for example, cGAMP, di-AMP, or di-GMP, but the present invention is not limited thereto.

The "cholesterol" used herein is a type of lipid, and is the generic term for steroid-based organic materials having a hydrophobic property, and the cholesterol may include various derivatives based on a cholesterol structure, and compounds that can be obtained by chemically changing a part of cholesterol. Preferably, the cholesterol includes bile acids (cholic acid, deoxycholic acid, lithocholic acid, and chenodeoxycholic acid), vitamin D, and steroid hormones (testosterone, estradiol, cortisol, aldosterone, prednisolone, and prednisone), but the present invention is not limited thereto. In addition, the cholesterol is a material that assists the toll-like receptor 7/8 agonist to be located on the surface and in various forms of nanoparticles, and may be replaced with lipid materials having a similar function thereto, for example, natural lipids such as a phospholipid, and synthetic lipids. Since the cholesterol serves to inactivate toll-like receptor 7 or 8 agonist when binding to its active site and prevents the toll-like receptor 7 or 8 agonist from being absorbed into a blood vessel in the body, there is no limitation as long as it is any type of well-known lipid.

The "cleavable linker" used herein includes a cleavable bond, and is the generic term for linkers in which cleavage can occur due to conditions such as low pH, enzymes or glutathione in the body such as a tumor microenvironment or the physiological environments of endosomes and lysosomes in cells; or external stimuli, that is, specific stimuli such as a temperature, redox potential, ultrasound, magnetic field, and near-infrared light. The cleavable linker preferably means a linker including a carbamate, disulfide, hydrazine, ester, peptide, or azide, but there is no limitation as long as it has a cleavable form. Examples of cleavable linkers include a linker group cleavable by enzymes including tobacco etch virus protease (TEV), trypsin, thrombin, cathepsin B, cathepsin D, cathepsin K, caspase 1, matrix metalloproteinase sequences, phosphodiester, phospholipid, ester, and beta-galactose; a linker group cleavable by nucleophilic/basic reagents including dialkyl dialkoxysilane, a cyanoethyl group, sulfone, ethylene glycolyl disuccinate, 2-N-acyl nitrobenzenesulfonamide, a-thiophenylester, unsaturated vinyl sulfide, sulfonamide after activation, malondialdehyde (MDA)-indole derivatives, levulinoyl ester, hydrazone, acylhydrazone, and alkyl thioester; a linker group cleavable by reducing agents including disulfide bridges and azo compounds; a linker group cleavable by oxidizing agents including vicinal diols, and selenium compounds; and a linker group cleavable by organometallic or metal catalysts including disulfide bridges and azo compounds. In addition, a linker group cleavable by electrophilic/acidic reagents includes a paramethoxybenzyl derivative, a tert-butylcarbamate analogue, dialkyl or diaryl dialkoxysilane, orthoester, acetal, aconityl, hydrazone, b-thiopropionate, phosphoramidate, imine, trityl, vinyl ether, polyketal, and alkyl 2-(diphenylphosphino)benzoate derivatives, and a linker group cleavable by photo-irradiation includes 2-nitrobenzyl derivatives, phenacyl ester, 8-quinolinyl benzenesulfonate, coumarin, phosphotriester, bis-arylhydrazone, and bimane bi-thiopropionic acid derivatives.

The "co-administration" used herein is administration of a toll-like receptor 7 or 8 agonist-cholesterol complex with various materials such as an antigen, an immune checkpoint inhibitor, an adjuvant, an immune activating material, and a chemotherapeutic agent, and there is no limitation to its type and form.

The term "chemotherapeutic agent" used herein refers to any compound known in the art that is used for cancer treatment without limitation, and for example, the chemotherapeutic agent includes, but not limited to, paclitaxel, docetaxel, 5-flurouracil, alendronate, doxorubicin, simvastatin, hydrazinocurcumin, amphotericin B, ciprofloxacin, rifabutin, rifampicin, efavirenz, cisplatin, theophyline, pseudomonas exotoxin A, zoledronic acid, trabectedin, siltuximab, dasatinib, sunitinib, apatinib, 5,6-dimethylxanthenone-4-acetic acid, silibinin, PF-04136309, trabectedin, carlumab, BLZ945, PLX3397, emactuzumab, AMG-820, IMC-CS4, GW3580, PLX6134, N-acetyl-l-cysteine, vitamin C, bortezomib, aspirin, salicylates, indolecarboxamide derivatives, quinazoline analogues, thalidomide, prostaglandin metabolites, 2ME2, 17-AAG, camptothecin, topotecan, pleurotin, 1-methylpropyl, 2-imidazolyl dissulphide, tadalafil, sildenafil, L-AME, nitroaspirin, celecoxib, NOHA, bardoxolone methyl, D,L-1-methyl-tryptophan, gemcitabine, axitinib, sorafenib, cucurbitacin B, JSI-124, anti-IL-17 antibodies, anti-glycan antibodies, anti-VEGF antibodies, bevacizumab, antracycline, tasquinimod, imatinib, and cyclophosphamide.

The term "immune checkpoint inhibitor" used herein refers to a cancer treatment method that activates the immune function of immune cells of the human body to fight cancer cells, and includes, but not limited to, anti-PD-1, anti-PD-L1, anti-CTLA-4, anti-KIR, anti-LAG3, anti-CD137, anti-OX40, anti-CD276, anti-CD27, anti-GITR, anti-TIM3, anti-41BB, anti-CD226, anti-CD40, anti-CD70, anti-ICOS, anti-CD40L, anti-BTLA, anti-TCR, and anti-TIGIT.

The term "antigen" used herein is the generic term for all materials causing immune responses in the body, and preferably include pathogens (bacteria, viruses, etc.), chemicals, pollen, cancer cells, shrimp, or some peptides or proteins thereof, and more preferably, tumor antigen peptides. However, there is no limitation as long as it is a material that can induce an immune response in the body. The antigen is preferably a protein, a recombinant protein, a glycoprotein, a gene, a peptide, a polysaccharide, a lipopolysaccharide, a polynucleotide, a cell, a cell lysate, a bacterium, or a virus, and more preferably, a tumor antigen peptide. The protein may be an antibody, an antibody fragment, a structural protein, a regulatory protein, a transcription factor, a toxin protein, a hormone, a hormone derivative, an enzyme, an enzyme fragment, a transport protein, a receptor, a receptor fragment, a bio-defense inducer, a storage protein, a movement protein, an exploitive protein, or a reporter protein. However, there is no limitation as long as it is a material acting as an antigen in the body to induce an immune response.

The "prevention" used herein refers to all actions that inhibit diseases such as infectious diseases, cancer, metabolic syndrome, autoimmune diseases, and rare diseases or delay the onset thereof by administration of the composition according to the present invention.

The "treatment" used herein refers to all actions that are involved in improving or beneficially changing symptoms of infectious diseases, cancer, metabolic syndrome, autoimmune diseases, or rare diseases by administration of the composition according to the present invention.

The "individual or subject" used herein refers to a target to which the composition of the present invention can be administered, and there is no limitation to the target.

The "infectious disease" used herein is the generic term for diseases induced by infection caused by a heterogenous organism, such as a virus, a bacterium or a fungus.

The "cancer" used herein is the generic term for various blood cancers, malignant solid tumors, etc. which can expand locally by infiltration and systemically by metastasis. Although not particularly limited, specific examples of cancer include colorectal cancer, adrenal cancer, bone cancer, brain cancer, breast cancer, bronchial cancer, colon and/or rectal cancer, gallbladder cancer, gastrointestinal cancer, head and neck cancer, kidney cancer, laryngeal cancer, liver cancer, lung cancer, nerve tissue cancer, pancreatic cancer, prostate cancer, parathyroid cancer, skin cancer, stomach cancer, and thyroid cancer. Other examples of cancer include adenocarcinoma, adenoma, basal cell carcinoma, cervical dysplasia and in situ carcinoma, Ewing's sarcoma, epidermoid carcinomas, giant cell tumors, glioblastoma multiforme, hairy-cell tumors, intestinal ganglioneuroma, hyperplastic corneal nerve tumors, islet cell carcinoma, Kaposi's sarcoma, leiomyoma, leukemias, lymphomas, malignant carcinoid, malignant melanomas, malignant hypercalcemia, marfanoid habitus tumors, medullary carcinoma, metastatic skin carcinoma, mucosal neuroma, myelodysplastic syndrome, myeloma, mycosis fungoides, neuroblastoma, osteosarcoma, osteogenic and other sarcoma, ovarian tumors, pheochromocytoma, polycythemia vera, primary brain tumors, small-cell lung tumors, squamous cell carcinoma of both ulcerating and papillary types, seminoma, soft tissue sarcoma, retinoblastoma, rhabdomyosarcoma, renal cell tumors or renal cell carcinoma, reticulum cell sarcoma, and Wilm's tumors. In addition, astrocytoma, gastrointestinal stromal tumors (GISTs), glioma or glioblastoma, hepatocellular carcinoma (HCC), and pancreatic neuroendocrine tumors are also included.

The "metabolic syndrome" used herein refers to a combination of three or more among five risk factors (high blood pressure, hyperglycemia, hypertriglyceridemia, low HDL cholesterol, and abdominal obesity) that increase health problems including heart disease, diabetes and stroke, occurring in one individual, and for example, includes metabolic diseases such as obesity, diabetes, high blood pressure, hyperlipidemia, heart disease, and gout. The metabolic syndrome is also the generic term for all diseases caused by metabolic syndrome.

The "autoimmune disease" used herein is the generic term for diseases caused by pathological responses to an autoantigen, and includes systemic autoimmune diseases such as systemic lupus erythematosus (SLE), rheumatoid arthritis (RA), and multiple sclerosis (MS), insulin-dependent diabetes mellitus (IDDM), Grave's disease, and allergies.

The "rare disease" used herein is the generic term for all diseases affecting only a small proportion of the population, which are usually hereditary, and generally refers to a disease that is difficult to diagnose due to its very low incidence or prevalence and has no proper treatment. Although each country has a slightly different definition, in Korea, in accordance with Article 2 of the Rare Disease Management Act, the rare disease is defined as "a disease in which the prevalence population is less than 20,000 or prevalence population is unknown because it is difficult to diagnose, determined according to the procedures and standards regulated by the ordinance of the Ministry of Health and Welfare." The World Health Organization (WHO) designates a rare disease as a case in which the prevalence is about 0.65 to 1 per 1,000 people, and the United States designates a rare disease as a case in which the total number of patients is less than 200,000, and Europe designates a rare disease as a case in which the total number of patients is 5 per 10,000 people.

The "pharmaceutical composition" used herein may be prepared in the form of a capsule, tablet, granule, injection, ointment, powder, or drink, and the pharmaceutical composition may be intended for humans. The pharmaceutical composition may be, but is not limited to, formulated in the form of an oral formulation such as a powder, granules, a capsule, a tablet or an aqueous suspension, a preparation for external use, a suppository and a sterile injectable solution according to a conventional method. The pharmaceutical composition of the present invention may include a pharmaceutically acceptable carrier. The pharmaceutically acceptable carrier may be a binder, a glidant, a disintegrant, an excipient, a solubilizer, a dispersant, a stabilizer, a suspending agent, a colorant, or a flavoring agent for oral administration, and for injection, a mixture of a buffer, a preservative, a pain relief agent, a solubilizer, an isotonic agent, and a stabilizer may be used, and for topical administration, a base, an excipient, a lubricant, and a preservative may be used. The pharmaceutical composition of the present invention may be prepared in various forms by being mixed with the above-described pharmaceutically acceptable carriers. For example, for oral administration, the pharmaceutical composition of the present invention may be prepared in various dosage forms such as a tablet, a troche, a capsule, an elixir, a suspension, a syrup and a wafer, and for injectables, the pharmaceutical composition of the present invention may be prepared in a unit dose ampoule or multiple dose forms. In addition, the pharmaceutical composition of the present invention may be formulated as a solution, a suspension, a tablet, a capsule or a sustained-release preparation.

Meanwhile, the carrier, excipient, and diluent for preparation may include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil. In addition, the carrier, excipient, and diluent for preparation may further include a filler, an anticoagulant, a lubricant, a wetting agent, a flavoring agent, an emulsifier, and a preservative.

The pharmaceutical composition according to the present invention is administered, but not limited to, orally, intravenously, intramuscularly, intraarterially, intramedullary, intrathecally, intracardially, transdermally, subcutaneously, intraperitoneally, intranasally, enterally, topically, sublingually, or rectally. Oral or parenteral administration is preferable. The term "parenteral" used herein means subcutaneous, intravenous, intramuscular, intraarticular, intrabursal, intrasternal, intrathecal, intralesional and intracranial injections or infusions. The pharmaceutical composition of the present invention may be administered in the form of a suppository for rectal administration.

The pharmaceutical composition of the present invention may vary according to various factors including the activity of a specific compound used, age, body weight, general health, sex, diet, administration time, administration route, excretion rate, drug formulation, and the severity of a specific disease to be prevented or treated, and the dose of the pharmaceutical composition may be selected by those of ordinary skill in the art according to a patient's condition and body weight, severity of a disease, a dosage form, an administration route and duration and may be administered daily at 0.0001 to 500 mg/kg or 0.001 to 500 mg/kg. The pharmaceutical composition of the present application may be administered once a day or several times in divided portions. The dose does not limit the scope of the present application in any way. The pharmaceutical composition according to the present invention may be formulated as a pill, a sugar-coated tablet, a capsule, a liquid, a gel, a syrup, a slurry or a suspension.

Hereinafter, to help in understanding the present invention, exemplary examples will be suggested. However, the following examples are merely provided to more easily understand the present invention, and not to limit the present invention.

### [Examples]

### Example 1: Method of synthesizing kinetically acting cholesterol-toll-like receptor agonist complex

To prepare a toll-like receptor agonist (TLR agonist) with kinetically controlled activity, which is present in an inactive form at the early stage, but is capable of exhibiting an immune activation efficacy by being converted to an activated state due to a physiological environment (low pH, enzyme, or glutathione) after being delivered into targeted cells such as a tumor microenvironment or immune cells in the body, complexs in which cholesterol binds to an amine (NH₂) moiety, i.e., the active site of various toll-like receptor 7 or 8 agonists (imidazoquinoloine-based agonists, 8-hydroxyadenine-based agonists, pteridone-based agonists, 2-aminopyrimidine-based agonists, benzoazepine-based agonists, and 7-thia-8-oxoguanosine-based agonists) were prepared by the chemical reaction of Scheme 1 or 2 below. The toll-like receptor 7 or 8 agonist was bound to cholesterol (Sigma-Aldrich) through a cleavable bond, such as a carbamate, disulfide, ester, peptide, or azide bond.

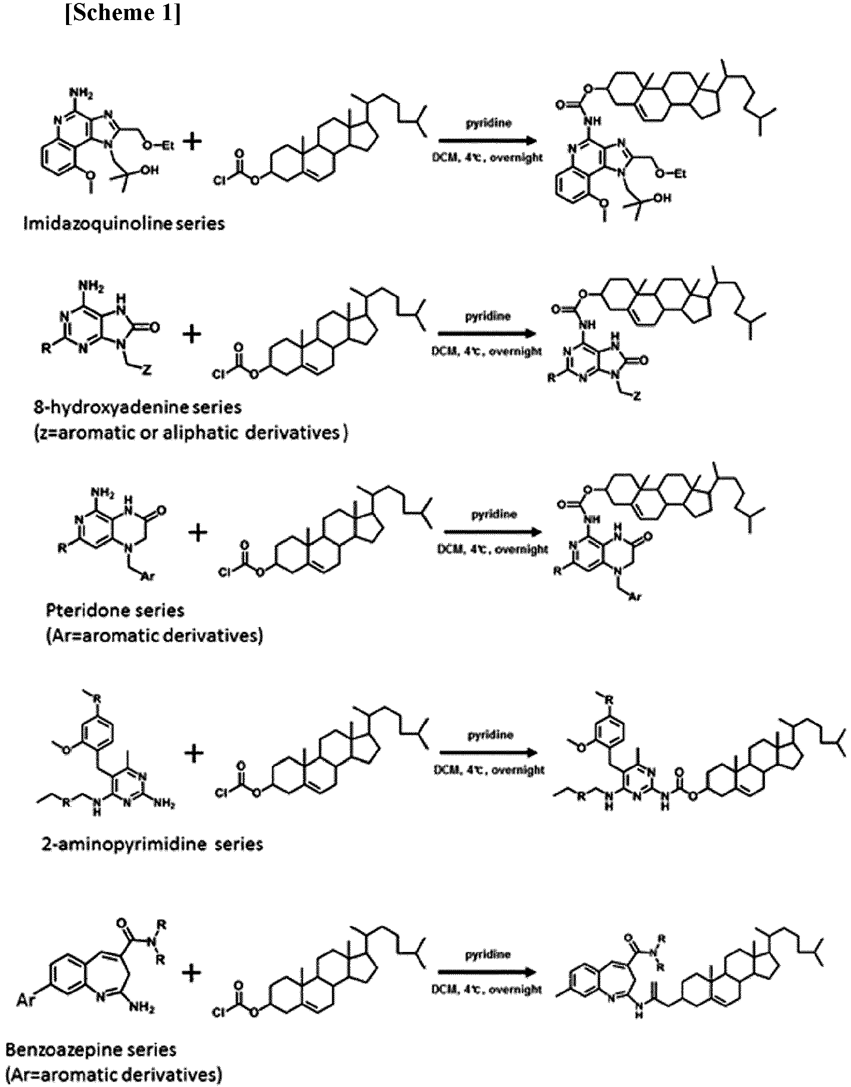

R is a side chain having an aliphatic or aromatic group, and may include - NH-, -CO-, -CONH-, -CSNH-, -COO-, -CSO-, -SO₂NH-, -SO₂-, -SO-, and -O-.

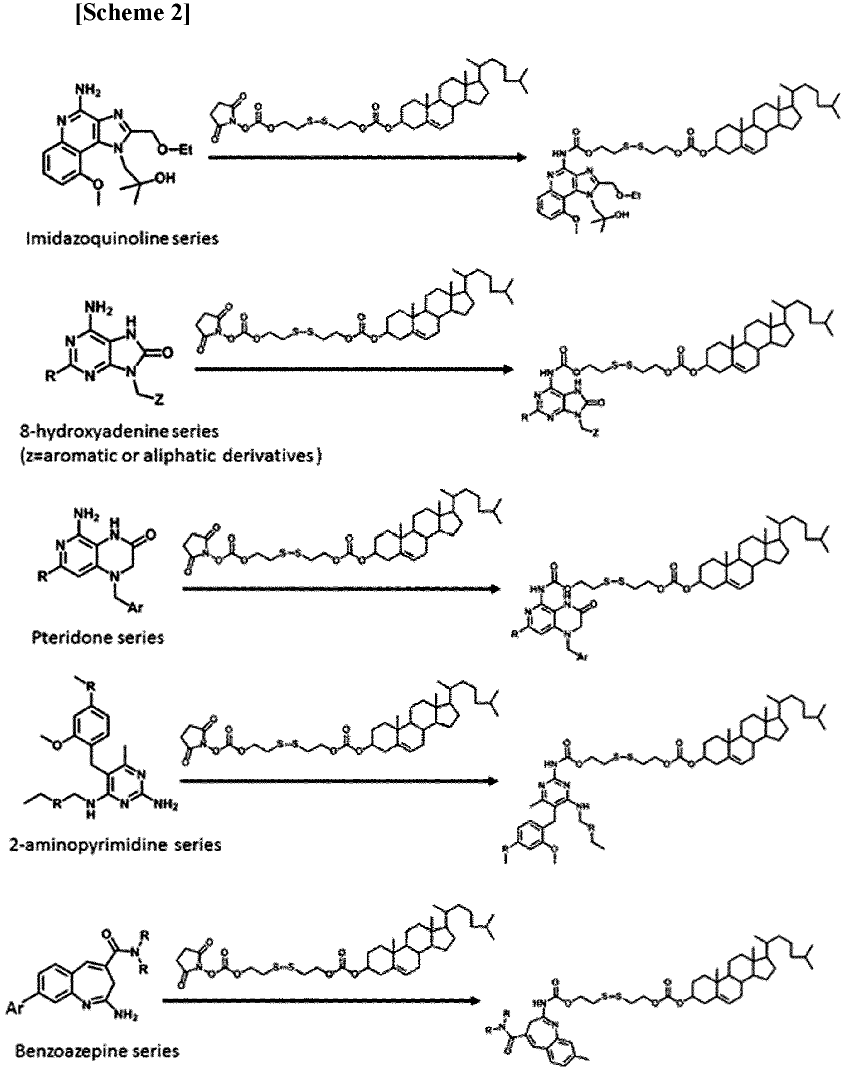

R is a side chain having an aliphatic or aromatic group, and may include - NH-, -CO-, -CONH-, -CSNH-, -COO-, -CSO-, -SO₂NH-, -SO₂-, -SO-, and -O-.

### Example 2: Synthesis of 2-methyl-1-(3-nitroquinolin-4-ylamino)propan-2-ol

2-Methyl-1-(3-nitroquinolin-4-ylamino)propan-2-ol (Compound 2) was synthesized using the method of Scheme 3 below. More specifically, 1-amino-2-methylpropan-2-ol (14 g) and tetraethylamine (9.6 g) were added to Compound 1 (30 g)-added dichloromethane (450 mL) at 10 to 20 °C and stirred for 2 hours, thereby preparing a mixture. Then, the mixture was concentrated by evaporating the solvent *in vacuo,* and then triturated with methyl tert-butyl ether (150 mL). The triturated mixture was separated using a filter and concentrated under low pressure, thereby obtaining Compound 2 (32 g, 85.2%, yellow solid). The structure of the obtained Compound 2 was verified using ¹H NMR.

¹H NMR (400 MHz, DMSO-d6): δ 9.91 (brs, 1H), 9.18 (s, 1H)), 8.46 (d, *J* = 8.0Hz, 1H), 7.83-7.92 (m, 2H), 7.56-7.60 (m, 1H), 5.15 (s, 1H), 3.86 (d, *J* = 4.8Hz, 2H), 1.15 (s, 6H).

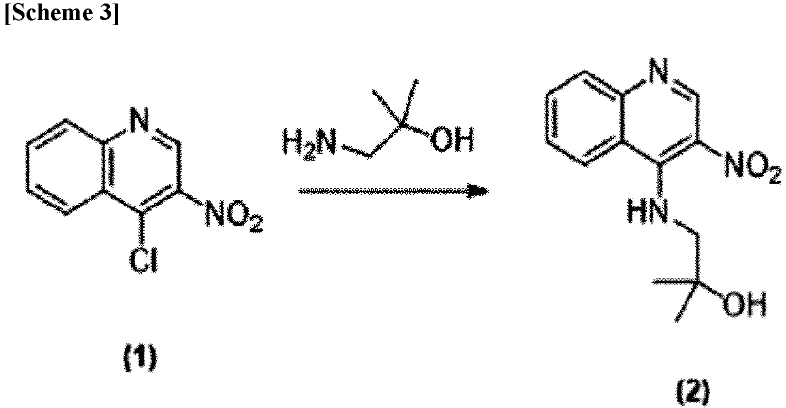

### Example 3: Synthesis of 1-(3-aminoquinolin-4-ylamino)-2-methylpropan-2-ol

1-(3-aminoquinolin-4-ylamino)-2-methylpropan-2-ol (Compound 3) was synthesized using the method of Scheme 4 below. In further detail, after mixing Compound 2 (32 g), methanol (500 mL) and Pd/C catalyst (3.2 g) in a reactor at 10 to 20 °C, the mixture was degassed and flushed three times using hydrogen. The hydrogen was vaporized to maintain 1 atm and the mixture was stirred at room temperature for 5 hours. Afterward, the mixture was triturated with methyl tert-butyl ether (100 mL). The triturated mixture was separated using a filter and concentrated at a low pressure, thereby obtaining Compound 3 (27 g, 95.4%, yellow solid). The structure of the obtained Compound 3 was verified using ¹H NMR.

¹H NMR (400 MHz, DMSO-d6): δ 8.37 (s, 1H)), 7.99-8.01 (m, 1H), 7.72-7.74 (m, 1H), 7.32-7.39 (m, 2H), 5.04 (s, 2H), 4.77 (brs, 1H), 4.67-4.70 (m, 1H), 4.12 (brs, 2H), 1.15 (s, 6H).

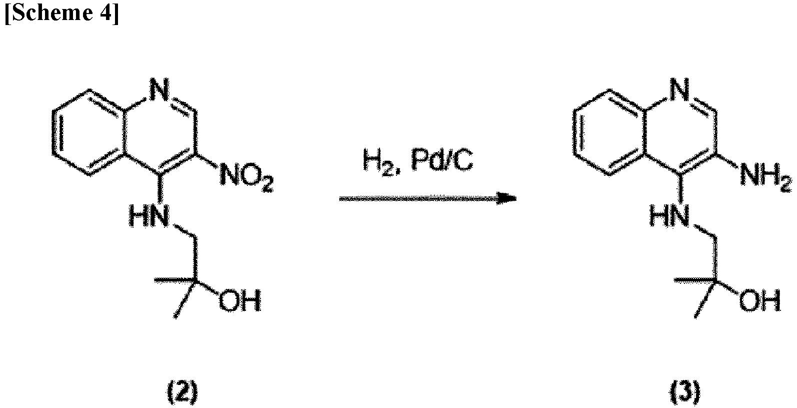

### Example 4: Synthesis of 1-(2-ethoxymethyl)-1H-imidazo[4,5-c]quinolin-1-yl)-2-methylpropan-2-ol

1-(2-ethoxymethyl)-1H-imidazo[4,5-c]quinolin-1-yl)-2-methylpropan-2-ol (Compound 4) was synthesized using the method of Scheme 5 below. In further detail, Compound 3 (27 g) and 2-ethoxyacetic acid (30 mL) were added to a reactor at 10 to 20 °C, and stirred at 120 to 130 °C for 5 hours. Afterward, the mixture was cooled to 20 to 25 °C, and then saturated sodium carbonate (150 mL) was added. Then, a reaction product was extracted using a mixed solution of dichloromethane and methanol (10/1, v/v). The extracted organic solution layer was washed with brine, and then moisture was removed over sodium sulfate (10 g). Then, the dehydrated organic solution layer was filtered using a filter and concentrated under low pressure, thereby obtaining Compound 4 (30 g, 85.8%, yellow gel). The structure of the obtained Compound 4 was verified using ¹H NMR.

¹HNMR (DMSO_d6 400 MHz): δ 9.18 (s, 1H)), 8.63 (d, *J* = 8.0Hz, 1H), 8.13 (dd, *J* = 1.6, 8.0Hz, 1H), 7.63-7.71 (m, 2H), 4.91 (s, 2H), 4.78 (brs, 2H), 3.54 (q, *J* = 6.8Hz, 2H), 1.10-1.18 (m, 9H).

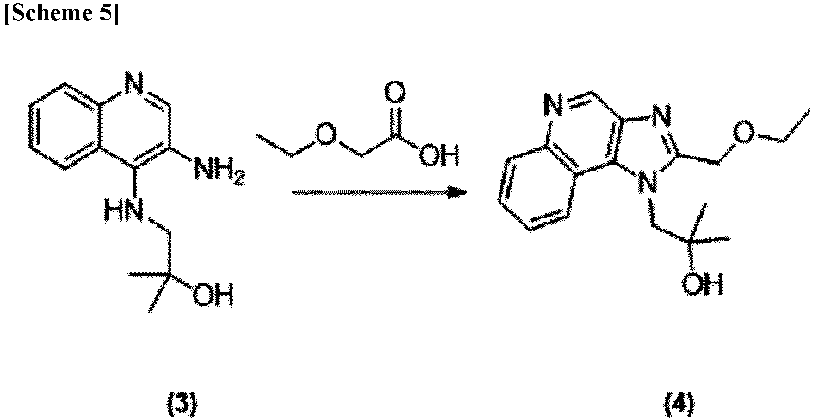

### Example 5: Synthesis of 2-(ethoxymethyl)-1-(2-hydroxy-2-methylpropyl)-1H-imidazo[4,5-c]quinolin 5-oxide

2-(ethoxymethyl)-1-(2-hydroxy-2-methylpropyl)-1H-imidazo[4,5-c]quinolin 5-oxide (Compound 5) was synthesized using the method of Scheme 6 below. In further detail, Compound 4 (30 g), dichloromethane (350 mL), and meta-chloroperoxybenzoic acid (26 g) were added to a reactor at 10 to 20 °C, and stirred at room temperature for 4 hours. Then, a saturated sodium carbonate solution (150 mL) and a sodium sulfate solution (150 mL) were added to the stirred mixture, and then a reaction product was extracted using a mixed solution of dichloromethane and methanol (10/1, v/v). The extracted organic solution layer was dehydrated over sodium sulfate (30 g), and filtered using a filter, followed by concentration under low pressure. Afterward, the concentrated reaction product was triturated using ethyl acetate (50 mL), separated using a filter, and then dried under low pressure, thereby obtaining Compound 5 (30 g, 94.9%, yellow solid). The structure of the obtained Compound 5 was verified using ¹H NMR.

¹HNMR (DMSO_d6 400 MHz): δ 9.04 (s, 1H)), 8.79 (d, *J* = 8.4Hz, 1H), 8.71 (d, *J* = 8.4Hz, 1H), 7.77-7.80 (m, 2H), 4.93 (s, 2H), 4.73 (brs, 2H), 3.54 (q, *J* = 6.8Hz, 2H), 1.12-1.18 (m, 9H).

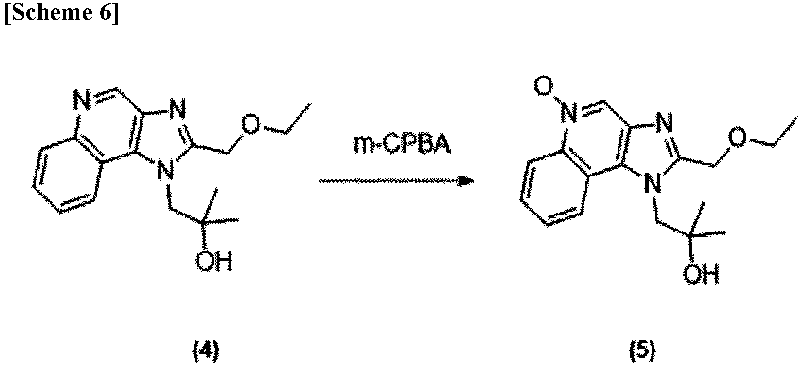

### Example 6: Synthesis of 1-(4-amino-2-ethoxymethyl)-1H-imidazo[4.5-c]quinolin-1-yl)-2-methylpropan-2-ol

1-(4-amino-2-ethoxymethyl)-1H-imidazo[4.5-c]quinolin-1-yl)-2-methylpropan-2-ol (Compound 6) was synthesized using the method of Scheme 7 below. In further detail, Compound 5 (30 g), DCM (600 mL), 4-methylbenzyl-1-sulfonyl chloride (18.2 g), and ammonia (NH₃.H₂O, 180 mL) were added to a reactor at 10 to 20 °C, and stirred at room temperature for 16 hours. Then, after adding distilled water to the stirred mixture, the mixture was separated using a mixed solution of dichloromethane and methanol (10/1, v/v). The separated organic solution layer was washed with brine and then dehydrated over anhydrous sodium sulfate (50 g). The dehydrated organic solution layer was filtered using a filter and then concentrated under low pressure, followed by triturating the concentrated reaction product using a mixed solution of methyl tert-butyl ether and methanol (15/1, v/v) for 30 minutes. Then, the resulting product was separated using a filter and dried under low pressure, thereby obtaining Compound 6 (18 g, 60%, yellow solid). The structure of the obtained Compound 6 was verified using ¹H NMR.

¹HNMR (DMSO_d6 400 MHz): δ 8.27 (d, *J* = 8.0Hz, 1H), 7.59 (d, *J* = 7.6Hz, 1H), 7.40 (t, *J* = 7.2Hz, 1H), 7.21 (t, *J* = 7.2Hz, 1H), 6.57 (brs, 2H), 4.89 (s, 2H), 4.68 (brs, 2H), 3.52 (q, *J* = 6.8Hz, 2H), 1.11-1.17 (m, 9H).

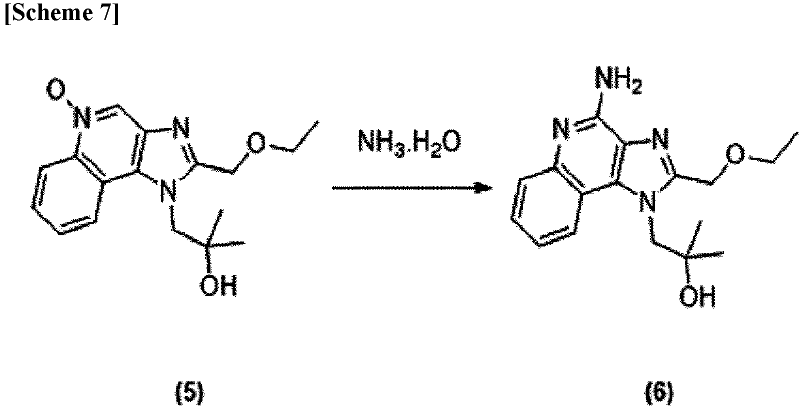

### Example 7: Synthesis of 10,13-dimethyl-17-(6-methylheptan-2-yl)-2,3,4,7,8,9,10,11,12,13,14,15,16,17-tetradecahydro-1H-cyclo[a]phenanthren-3-yl 2-(ethoxymethy)-1-(2-hydroxy-2-methylpropyl)-1H-imidazo[4,5-c]quinolin-4-yl carbamate

10,13-dimethyl-17-(6-methylheptan-2-yl)-2,3,4,7,8,9,10,11,12,13,14,15,16,17-tetradecahydro-1H-cyclo[a]phenanthren-3-yl 2-(ethoxymethyl)-1-(2-hydroxy-2-methylpropyl)-1H-imidazo[4,5-c]quinolin-4-yl carbamate (Compound 8) was synthesized using the method of Scheme 8 below. First, Compound 7 (TCI, 50 g, cholesterol chloroformate) was subjected to 250g-silica gel-filled column chromatography (0%~20% ethyl acetate in n-hexane), thereby obtaining pure Compound 7 (30 g). Then, Compound 6 (15 g) and dichloromethane (198.9 g) were added to a reactor at 10 to 20 °C, and the pure Compound 7 (30 g) and tetraethylamine (9.6 g) were sequentially added, followed by stirring at 20 to 25 °C for 16 hours. After adding water to the stirred mixture, dichloromethane was added to extract a reaction product. The extracted organic solution layer was washed with brine and dehydrated over anhydrous sodium sulfate (195 g). The dehydrated organic solution layer was filtered using a filter, and concentrated under low pressure. Then, the concentrated reaction product was triturated using a mixed solution of methyl tert-butyl ether and methanol (10/1, v/v). Afterward, Compound 8 (10.2 g, 55.1%, white solid) was obtained by separation using a filter and drying under low pressure. The structure of the obtained Compound 8 was verified using ¹H NMR. From the ¹H NMR result, it was confirmed that a complex in which resiquimod (R848) and cholesterol are connected via a carbamate bond was prepared.

¹H NMR (CDCl₃ 400 MHz): δ 8.13-8.19 (m, 2H), 7.59-7.63 (m, 1H)), 7.46-7.50 (m, 1H), 5.42-5.43 (m, 1H), 4.92 (brs, 2H), 4.72-4.80 (m, 3H), 3.68 (q, J = 6.8Hz, 2H), 3.24 (s, 1H), 2.51-2.59 (m, 1H), 2.36-2.47 (m, 1H), 1.96-2.11 (m, 3H), 1.81-1.95 (m, 2H), 1.45-1.75 (m, 9H), 1.02-1.35 (m, 27H), 0.94 (d, J = 6.4Hz, 3H), 0.89 (d, J = 6.4Hz, 6H), 0.71 (s, 3H).

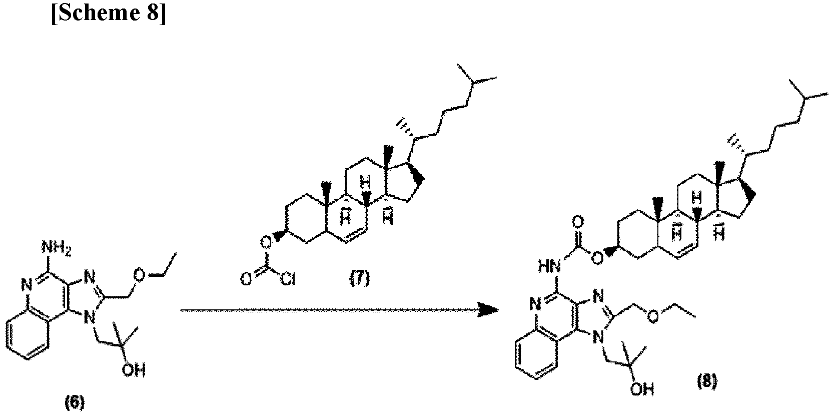

### Example 8: Synthesis of bis(2,5-dioxopyrrolidin-1-yl) 2,2'-disulfanedivlbis(ethane-2,1-divl) dicarbonate

Bis(2,5-dioxopyrrolidin-1-yl) 2,2'-disulfanediylbis(ethane-2,1-diyl) dicarbonate (Compound 9) was synthesized using the method of Scheme 9 below. First, Compound 7 (TCI, 70 g) was subjected to 350 g-silica gel-filled column chromatography (0%~20% ethyl acetate in n-hexane), thereby obtaining pure Compound 7 (40 g). Then, the pure Compound 7 (40 g) and dichloromethane (100 mL) were added to a reactor at 10 to 15 °C, and a bis(2-hydroxyethyl) disulfide-added dichloromethane (25 0mL) solution and pyridine (21 g) were sequentially added. The mixture was stirred at room temperature for 2 hours, and then distilled water (200 mL) was added. Subsequently, dichloromethane (150 mL) was added three times, thereby extracting a reaction product. The extracted organic solution layer was washed with brine and dehydrated over anhydrous sodium sulfate (20 g). The dehydrated reaction product was filtered using a filter, and then concentrated under low pressure. Afterward, Compound 9 (20 g, 39.6%, yellow gel) was obtained using column chromatography (silica gel, 300 g, 10%~30% ethyl acetate in n-hexane). The structure of the obtained Compound 9 was verified using ¹H NMR.

¹HNMR (CDCl₃ 400 MHz): δ 5.41-5.42 (m, 1H), 4.46-4.56 (m, 1H), 4.41 (t, *J* = 6.8Hz, 2H), 3.91 (t, *J* = 6.0Hz, 2H), 2.98 (t, *J* = 6.8Hz, 2H), 2.91 (t, *J* = 6.0Hz, 2H), 2.35-2.47 (m, 2H), 1.79-2.08 (m, 6H), 1.43-1.73 (m, 7H), 1.25-1.42 (m, 5H), 1.06-1.22 (m, 7H), 0.97-1.03 (m, 5H), 0.93 (d, *J =* 6.4Hz, 3H), 0.88 (dd, *J =* 1.6, 6.8Hz, 6H), 0.69 (s, 3H).

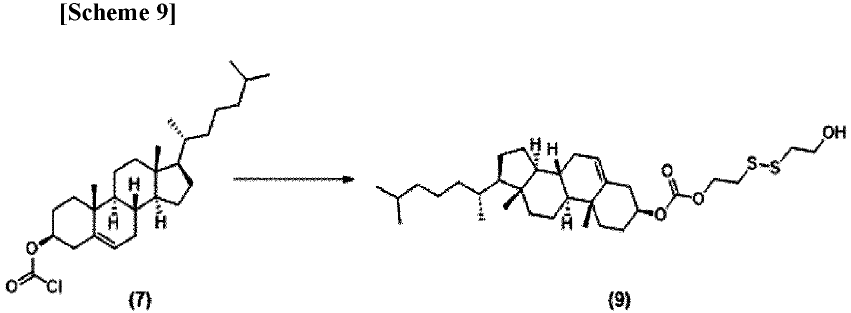

### Example 9: Synthesis of Compound 10

Compound 10 was synthesized using the method of Scheme 10 below. In further detail, Compound 9 (20 g) and dichloromethane (200 mL) were added to a reactor at 10 to 15 °C, and bis(2,5-dioxopyrrolidin-1-yl) carbonate (18 g) and tetraethylamine (10.7 g) were sequentially added. The mixture was stirred at room temperature for 3 hours, distilled water (300 mL) was added, and dichloromethane (150 mL) was then added three times to extract a reaction product. The extracted organic solution layer was washed with brine and dehydrated using anhydrous sodium sulfate (20 g). The dehydrated reaction product was filtered using a filter, and then the filtrate was concentrated under low pressure. Afterward, Compound 10 (18 g, 72%, yellow gel) was obtained using column chromatography (silica gel, 200 g, 5%~20% ethyl acetate in n-hexane). The structure of the obtained Compound 10 was verified using ¹H NMR.

¹HNMR (CDCl₃ 400 MHz): δ 5.39-5.40 (m, 1H), 4.57 (t, *J* = 6.8Hz, 2H), 4.43-4.52 (m, 1H), 4.37 (t, *J* = 6.4Hz, 2H), 2.96-3.03 (m, 4H), 2.84 (s, 4H), 2.34-2.44 (m, 2H), 1.78-2.04 (m, 5H), 1.42-1.73 (m, 7H), 1.22-1.40 (m, 5H), 1.06-1.20 (m, 7H), 0.95-1.04 (m, 5H), 0.91 (d, *J* = 6.0Hz, 3H), 0.86 (d, *J* = 6.4Hz, 6H), 0.67 (s, 3H).

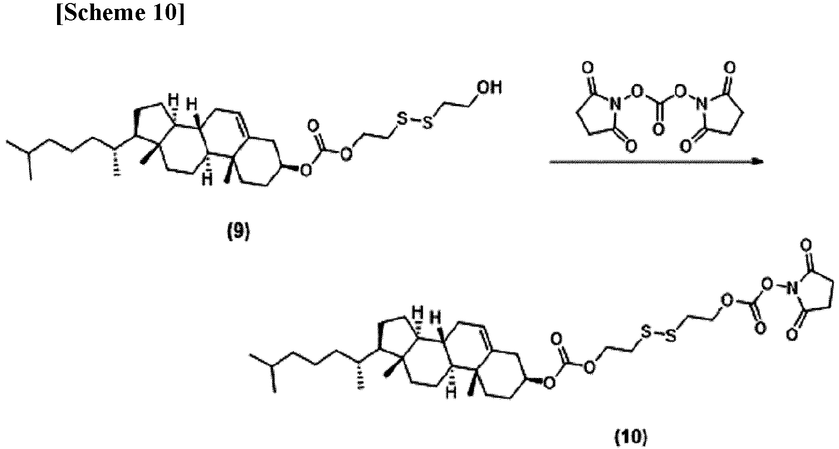

### Example 10: Synthesis of 2-((2-((10,13-diemthyl-17-(6-methylheptan-2-yl)-2,3,4,7,8,9,10,11,12,13,14,15,16,17-tetradecahydro-1H-cyclo[a]phenanthren-3-yloxy)cabonyloxy)ethyl)disulfanyl)ethyl 2-(ethoxymethyl)-1-(2-hydroxy-2-methylpropyl)-1H-imidazo[4,5-c]quinolin-4-yl carbamate

2-((2-((10,13-dimethyl-17-(6-methylheptan-2-yl)-2,3,4,7,8,9,10,11,12,13,14,15,16,17-tetradecahydro-1H-cyclo[a]phenanthren-3-yloxy)carbonyloxy)ethyl)disulfanyl)ethyl 2-(ethoxymethyl)-1-(2-hydroxy-2-methylpropyl)-1H-imidazo[4,5-c]quinolin-4-yl carbamate (Compound 11) was synthesized using the method of Scheme 11 below. In further detail, Compound 6 (15 g) and dichloromethane (198.9 g) were added to a reactor at 10 to 20 °C, and Compound 10 (40.5 g) and tetraethylamine (9.6 g) were sequentially added. The mixture was stirred at 20 to 25 °C for 16 hours, and then distilled water (225 mL) was added. Subsequently, dichloromethane (99.45 g) was added five times to extract a reaction product. The extracted organic solution layer was washed with brine and dehydrated over anhydrous sodium sulfate (195 g). Then, the dehydrated reaction product was filtered using a filter, and then the filtrate was concentrated under low pressure. Afterward, Compound 11 (10.8 g, 37.4%, white solid) was obtained using column chromatography (silica gel, 100 g, 10%~50% ethyl acetate in n-hexane). The structure of the obtained Compound 11 was verified using ¹H NMR. From the ¹H NMR result, it was confirmed that a complex in which R848 and cholesterol are cross-linked via disulfide was prepared.

¹H NMR (CDCl₃ 400 MHz): δ 8.15-8.17 (m, 2H), 7.60-7.64 (m, 1H)), 7.47-7.51 (m, 1H), 5.39-5.40 (m, 1H), 4.93 (s, 2H), 4.81 (s, 2H), 4.56 (t, *J* = 6.4Hz, 2H), 4.45-4.54 (m, 1H), 4.41 (t, *J* = 6.4Hz, 2H), 3.68 (q, *J* = 6.8Hz, 2H), 3.13 (s, 1H), 3.09 (t, *J* = 6.4Hz, 2H), 3.01 (t, *J* = 6.4Hz, 2H), 2.34-2.47 (m, 2H), 1.92-2.06 (m, 3H), 1.79-1.90 (m, 2H), 1.23-1.72 (m, 21H), 1.06-1.21 (m, 7H), 0.96-1.05 (m, 5H), 0.93 (d, *J* = 6.4Hz, 3H), 0.88 (dd, *J* = 1.6, 6.4Hz, 6H) , 0.69 (s, 3H).

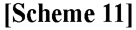

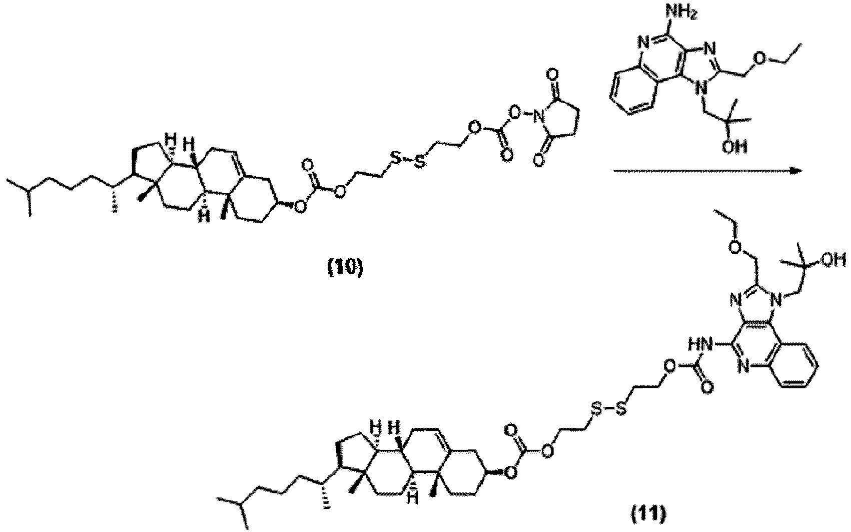

### Example 11: Preparation of nanoparticle including cholesterol-toll-like receptor 7 or 8 agonist complex

Since a cholesterol-toll-like receptor 7 or 8 agonist complex includes cholesterol, it may be easily prepared in various nanoparticle forms, and thus the interaction with immune cells may be maximized.

### 11.1. Preparation of nanoliposomes including cholesterol-conjugated toll-like receptor 7 or 8 agonist

To prepare a nanoliposome including cholesterol-conjugated toll-like receptor 7 or 8 agonists, a cholesterol-resiquimod complex, which is one of the cholesterol-toll-like receptor 7 or 8 agonist complexs, prepared in the same manner as in Example 1 was used. In further detail, 5 mg of 1,2-dioleoyl-sn-glycero-3-phosphocholine (DOPC, Avanti), 1.5 mg of dimethyldioctadecylammonium bromide (DDAB, Avanti), and 1 mg of the cholesterol-resiquimod complex were dissolved in 0.5 mL of chloroform, thereby preparing a mixture. The chloroform was evaporated from the mixture using a rotary evaporator (30 min), thereby preparing a multi-layered thin film, and 2 mL of phosphate buffered saline (PBS) was added and stirred at room temperature for 2 hours. Then, a single-layered liposome was obtained through homogenization using a tip sonicator (amplitude: 20%, 2 min), and then subjected to a mini extruder for homogenization of the nanoliposome, thereby preparing a nanoliposome including a stable cholesterol-toll-like receptor 7 or 8 agonist complex. The amount of cholesterol-toll-like receptor 7 or 8 agonist complex in the prepared nanoliposome was quantified using a UV-Vis spectrophotometer.

### 11.2. Preparation of nanoemulsion including cholesterol-conjugated toll-like receptor 7 or 8 agonist

To prepare a nanoemulsion including cholesterol-conjugated toll-like receptor 7 or 8 agonists, a cholesterol-resiquimod complex, which is one of the cholesterol-toll-like receptor 7 or 8 agonist complexs, prepared in the same manner as in Example 1 was used. In further detail, 1 mg of DOPC, 240 µg of cholesterol (Sigma-Aldrich), and 240 µg of the cholesterol-resiquimod complex were added to 1 mL of chloroform and dissolved, thereby preparing a mixture. Subsequently, the mixture was transferred to a round-bottom flask, and chloroform was completely evaporated using a rotary evaporator, thereby preparing a thin film. Then, squalene (5%v/v), Tween 80 (0.5%v/v), and Span 85 (0.5%v/v) were added to 2 mL of phosphate-buffered saline and dissolved, and then the resulting solution was added onto a lipid membrane and dispersed using a tip sonicator for 1 minute, stirred using a tube revolver for 2 hours, thereby preparing a nanoemulsion including a cholesterol-toll-like receptor 7 or 8 agonist complex. The prepared nanoemulsion was stored in a refrigerator at 4 °C before use.

### 11.3. Preparation of nanomicelle consisting of cholesterol-conjugated toll-like receptor 7 or 8 agonist and saponin

To prepare a nanomicelle including cholesterol-toll-like receptor 7 or 8 agonist complexs and saponins, a cholesterol-resiquimod complex, which is one of the cholesterol-toll-like receptor 7 or 8 agonist complexs, prepared in the same manner as in Example 1 was used. In further detail, phosphatidylcholine : saponin : cholesterol-resiquimod complex were mixed at a weight ratio of 5 : 3 : 2, and then added to ether and dissolved to have a concentration of 14 mg/mL, thereby preparing an ether solution containing a lipid. Subsequently, saponin was dissolved in 4 mL of distilled water at a concentration of 1.5 mg/mL, the resulting solution was placed in a 20 mL glass bottle, and the bottle was closed with a rubber plug and then stored in a water jacket at 55 °C. Then, 1 mL of a lipid-containing ether solution was added to the saponin-containing glass bottle at a rate of 0.2 mL/min using a syringe pump and stirred for 2 hours. Here, the tip of a syringe needle was located below the surface of the saponin-containing aqueous solution, and for ventilation, a second needle was inserted into the rubber stopper. Then, after moving the glass bottle to room temperature and stabilizing it by stirring for 3 days, a nanomicelle consisting of cholesterol- resiquimod complex and saponin was prepared.

### 11.4. Preparation of polymeric nanoparticle consisting of cholesterol-cojugated toll-like receptor 7 or 8 agonist

To prepare a polymeric nanoparticle including cholesterol-toll-like receptor 7 or 8 agonist complexs, a cholesterol-resiquimod complex, which is one of the cholesterol-toll-like receptor 7 or 8 agonist complexs, prepared in the same manner as in Example 1 was used. In further detail, 60 mg of PLGA polymer (Eudragit) having a composition ratio of lactide and glycolide of 50 : 50 was dissolved in 1 ml of a chloroform solvent. Subsequently, 5 mg of the cholesterol-resiquimod complex was added to the solvent, and the cholesterol-resiquimod complex and the polymer were dissolved using an ultrasonic bath (Emerson Model CPX5800H-E). Then, 200 µl of the dissolved solution was added to 10 mL of a 2.5% PVA aqueous solution, and dispersed for 1 minute using a tip sonicator (Sonics&Materials Model VCX 750). Here, the output of the sonicator was set to 750 watts, the vibration intensity was set to 20 kHz, and the amplitude was set to 20%. Then, to completely evaporate the PLGA-dissolved organic solvent, the prepared aqueous solution was stirred at 600 rpm and room temperature for more than 8 hours. To remove the unreacted polymer and cholesterol-resiquimod complex, centrifugation was performed using a centrifuge (Hanil, Combi-514R) at 12,000 rpm for 12 minutes, and after removing a supernatant, 10 mL of deionized water was added and then dispersed in a sonicator for 30 seconds. The above procedure was repeated three times, and then freeze drying was performed, followed by storing at -20 °C.

### Example 12: Confirmation of effect of cleaving chemical bond of kinetically acting cholesterol-toll-like receptor 7 or 8 agonist complex by γ-interferon-inducible lysosome thiol reductase present in endolysosome

### 12.1. Endocytosis of nanoliposome including kinetically acting cholesterol-toll-like receptor 7 or 8 agonist complex

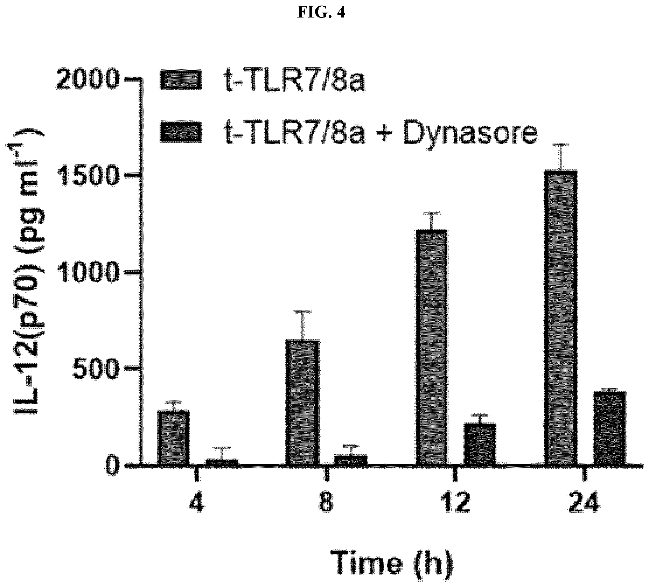

To confirm whether a nanoliposome including a complex in which cholesterol and resiquimod are conjugated by a disulfide bond, prepared in the same manner as in Example 11.1, moves into cells through endocytosis, an experiment was conducted using bone marrow-derived dendritic cells (BMDCs). In further detail, BMDCs were treated with Dynasore (40 µg) inhibiting endocytosis, and incubated for 1 hour. Then, 4, 8, 12, and 24 hours after treatment of the nanoliposome including a cholesterol-toll-like receptor 7 or 8 agonist complex, each cell culture was obtained. Then, the obtained cell culture was centrifuged at 1,500 rpm for 3 minutes to separate cells and a supernatant, and then the level of interleukin-12 (IL-12) included in the supernatant was measured by enzyme-linked immunosorbent assay (ELISA). All subsequent experiments were repeated at least three times, and the result was expressed as mean ± standard deviation. Statistical significance was confirmed by the Student's t-test, and when P < 0.05, it was determined that there is statistical significance. The result is shown in FIG. 4.

As shown in FIG. 4, it was confirmed that, in the group treated with an endocytosis inhibitor, a very low amount of IL-12 is secreted. From the result, it can be confirmed that the nanoliposome including a cholesterol-toll-like receptor 7 or 8 agonist complex normally moves into cells to exhibit an immune activation response.

### 12.2. Confirmation of expression of γ-interferon-inducible lysosomal thiol reductase in BMDCs and macrophages

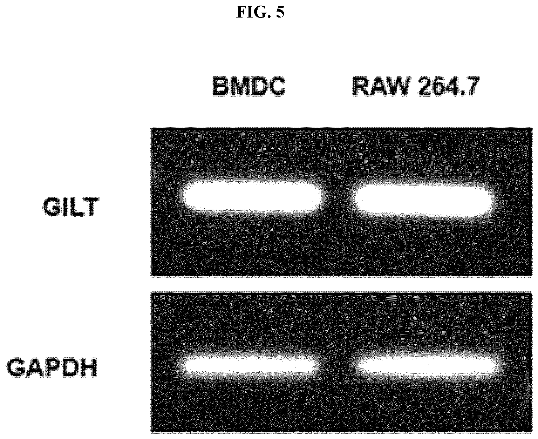

To confirm whether γ-interferon-inducible lysosomal thiol reductase (GILT) is expressed in BMDCs and macrophages, experiments were conducted using BMDCs and macrophages, that is, RAW 264.7 cells. In further detail, total ribonucleic acids (RNAs) of the BMDCs and the RAW 264.7 cells were isolated using Trizol reagent. Subsequently, the isolated RNA was used as a template to synthesize complementary deoxyribonucleic acid (cDNA) using a Maxime RT PreMix tool. Then, real-time polymerase chain reaction (RT-PCR) was performed using the synthesized cDNA, and a PCR product was subjected to agarose gel electrophoresis to confirm an expression level of GILT. The result is shown in FIG. 5.

As shown in FIG. 5, it was confirmed that GILT is expressed in dendritic cells and macrophages, which are antigen-presenting cells.

### 12.3. Confirmation of cleavage of chemical bond of kinetically-acting cholesterol-toll-like receptor 7 or 8 agonist complex by GILT

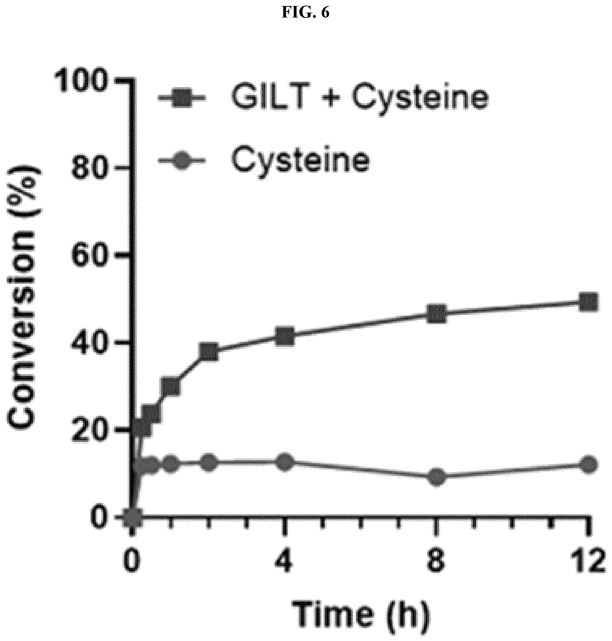

To confirm whether the disulfide bond, which is a chemical bond, between cholesterol and a toll-like receptor 7 or 8 agonist is cleaved by GILT present in an endolyososome, 50 µL of a nanoliposome including a complex in which cholesterol and resiquimod are conjugated by a disulfide bond, prepared in the same manner as in Example 11.1, was put in a 5 mL tube. Subsequently, cysteine dissolved in PBS with or without GILT was mixed with the nanoliposome and incubated at 37 °C in a shaking incubator, thereby obtaining samples over time. Then, the obtained samples were rapidly frozen using liquid nitrogen, and stored in a freezer at -20 °C. All samples obtained after 12 hours were freeze-dried in a vacuum freeze dryer at 10 Pa and -80 °C for 24 hours. Then, the freeze-dried sample was analyzed by liquid chromatography-mass spectrometry (LC-MS) to quantify the level of resiquimod (R848) separated from the cholesterol-toll-like receptor 7 or 8 agonist complex. The result is shown in FIG. 6.

As shown in FIG. 6, it was confirmed that the disulfide bond between cholesterol and R848 is cleaved by GILT and R848 is detected in a separated form.

From the results, it was confirmed that the nanoliposome including a complex in which cholesterol and a toll-like receptor 7 or 8 agonist are conjugated by a disulfide bond moves into cells through endocytosis, and the bond is cleaved by GILT present in an endolysosome in cells to separate cholesterol and the toll-like receptor 7 or 8 agonist, thereby exhibiting an immune activation action.

### Example 13: Confirmation of immune response induction effect by kinetically acting cholesterol-toll-like receptor 7 or 8 agonist complex

### 13.1. Confirmation of kinetically controlled activation of immune cells by nanoliposome including cholesterol-toll-like receptor 7 or 8 agonist complex

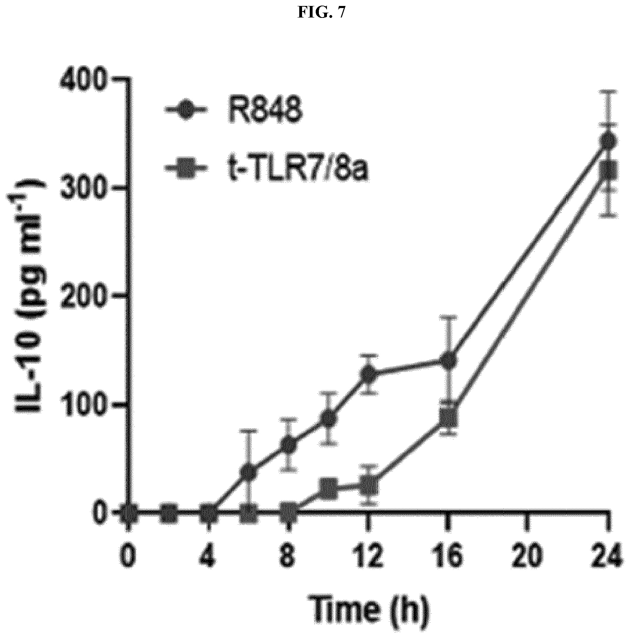

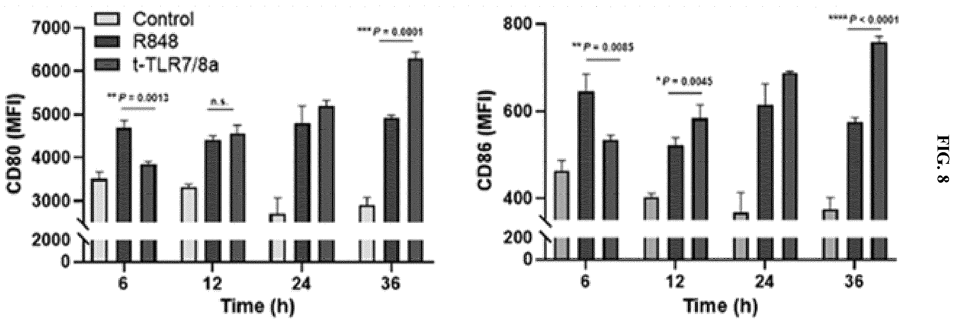

To confirm the effect of a nanoliposome including a complex in which cholesterol and resiquimod are conjugated by a disulfide bond, prepared in the same manner as in Example 11.1, on immune function modulation of immune cells, after treatment of BMDCs with the nanoliposome, the amount of cytokine secretion and the expression of co-stimulatory molecules over time were confirmed. More specifically, 1×10⁶ BMDCs were treated with the nanoliposome or R848 to have a toll-like receptor 7 or 8 agonist concentration of 1 µg/mL, and after four hours, a cell culture was obtained at four-hour intervals. The obtained cell culture was centrifuged at 1,500 rpm for 3 minutes to separate cells and a supernatant, and then amounts of IL-10 and IL-12 secretion included in the supernatant were measured using ELISA. The result is shown in FIG. 7. Then, the obtained cells were stained with fluorescence-tagged antibodies to confirm the degree of cell maturation, and then the expression of co-stimulatory molecules in dendritic cells was confirmed using a BD Canto II flow cytometer. The result is shown in FIG. 8.

As shown in FIG. 7, compared to an experimental group treated with R848 alone, in an experimental group treated with a nanoliposome including a cholesterol-R848 complex (t-TLR7/8a), it was confirmed that IL-10 secretion is delayed by at least 4 hours. It was also confirmed that the cytokine level continuously increases even after 24 hours.

As shown in FIG. 8, as a result of confirming the expression of co-stimulatory molecules indicating the degree of dendritic cell maturation, such as CD80 and CD86, in the experimental group treated with R848 alone, the expression level of co-stimulatory molecules indicating the degree of maturation does not increase over time, but in the experimental group treated with the nanoliposome including the cholesterol-R848 complex (t-TLR7/8a), it was confirmed that the expression level of co-stimulatory molecules increases over time.

From the results, it was confirmed that the cholesterol-toll-like receptor 7 or 8 agonist complex is a kinetically-controllable immune function modulator, and can induce not only an immune response after approximately 4 hours but also a continuous immune response. Then, to this end, it was confirmed that the cholesterol-toll-like receptor 7 or 8 agonist complex can maintain dendritic cells in a matured state, that is, a state that can induce an immune response for a long time, not in an exhausted state, that is, a state that does not induce an immune response.

### 13.2. Confirmation of continuous immune response-inducing effect by nanoliposome including kinetically acting cholesterol-toll-like receptor 7 or 8 agonist complex

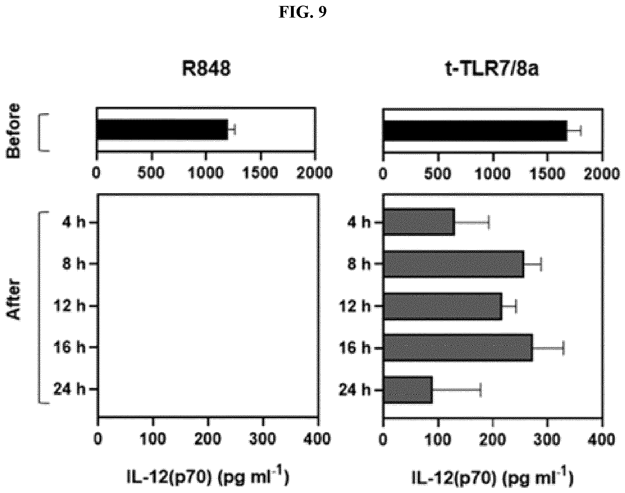

To confirm whether a nanoliposome including the complex in which cholesterol and resiquimod are conjugated by a disulfide bond, prepared in the same manner as in Example 11.1, continuously induces an immune response, 1×10⁶ of BMDC cells were treated with the nanoliposome or R848 to have a toll-like receptor 7 or 8 agonist concentration of 1 µg/mL, and 12 hours later, a cell culture was obtained. The obtained cell culture was centrifuged at 1,500 rpm for 3 minutes to separate cells and a supernatant, and then the amount of IL-12 secretion was measured using the obtained supernatant. Then, the obtained cells were seeded again in a fresh medium, and cell supernatants were obtained at four-hour intervals to check the amount of IL-12 secretion through ELISA. The result is shown in FIG. 9.

As shown in FIG. 9, as a result of culturing again after replacing with a fresh medium, in the R848-treated control, IL-12 was no longer observed, but in the nanoliposome-treated experimental group (t-TLR7/8a), IL-12 secretion continued for up to 16 hours. From the result, when the toll-like receptor 7 or 8 agonist was treated alone, it rapidly induces the depletion of dendritic cells, but the cholesterol-toll-like receptor 7 or 8 agonist complex maintains the maturation state of dendritic cells for a long time, inducing a continuous immune response.

### 13.3. Confirmation of effect of polarization-inducing effect of CD4-positive T cells to Th1 response by nanoliposome including kinetically acting cholesterol-toll-like receptor 7 or 8 agonist complex

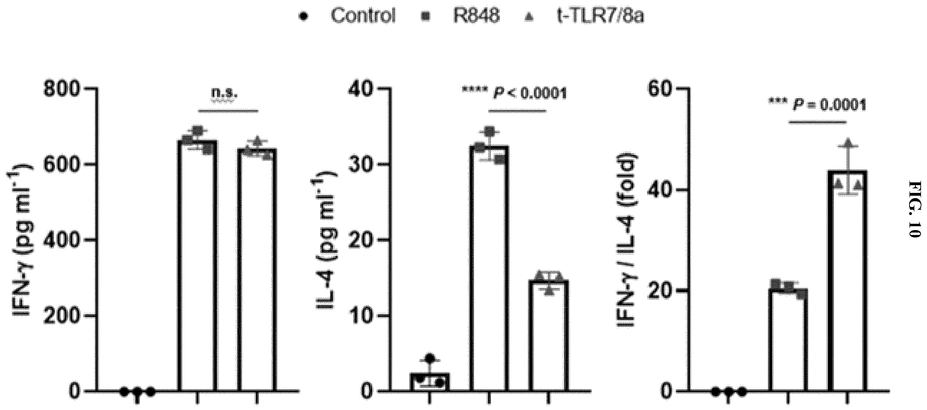

To confirm whether the polarization of CD4⁺ T cells to Th1 response is induced by the continuous immune response of a nanoliposome including a cholesterol-toll-like receptor 7 or 8 agonist complex, first, the spleen was taken from a C57BL/6 mouse to prepare single cells, and CD4⁺ T cells were isolated using a CD4⁺ T cell isolation kit. In addition, 1×10⁶ BMDC cells were treated with the nanoliposome or R848, together with OVA (10 µg/mL), to have a toll-like receptor 7 or 8 agonist concentration of 1 µg/mL, and incubated for 12 hours. Afterward, BMDCs and CD4⁺ T cells were seeded in 96-well plates at a ratio of 1 : 10, and cultured together. After 5 days culture, a culture supernatant was obtained, and the amounts of IL-4 and IFN-γ secretion were measured through ELISA. The result is shown in FIG. 10.

As shown in FIG. 10, it was confirmed that the amount of IFN-γ secretion did not show a significant difference by comparing the control treated with R848 and OVA (R848) and the experimental group treated with the nanoliposome and OVA (t-TLR7/8a), and the amount of IL-4 secretion is reduced in the experimental group treated with the nanoliposome and OVA (t-TLR7/8a). From the result, it was confirmed that IL-12 secretion was promoted in BMDCs treated with the nanoliposome including a cholesterol-toll-like receptor 7 or 8 agonist complex, and thus the CD4⁺T cells are polarized into Th1, thereby increasing the IFN-y/IL-4 ratio.

### Example 14: Confirmation of synergistic effect by co-administration of adjuvant

### 14.1. Confirmation of synergistic effect by combination of toll-like receptor 3 agonist and nanoliposome including kinetically acting cholesterol-toll-like receptor 7 or 8 agonist complex

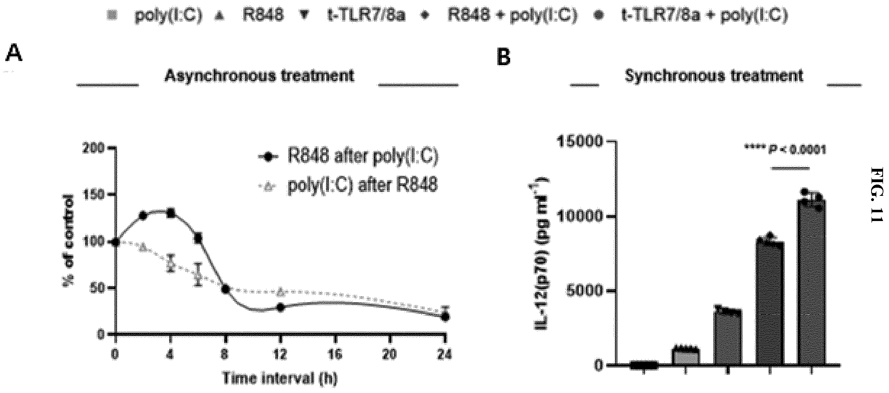

To confirm the synergistic effect caused by administration of a combination of adjuvants, BMDCs were treated with R848 and poly(I:C), which are toll-like receptor 3 agonists known as adjuvant. In further detail, asynchronous treatment was performed on experimental groups, and specifically, an experimental group in which BMDCs were treated first with poly(I:C) and then treated with the toll-like receptor 7 or 8 agonist R848, after a period of time (R848 after poly(I:C)), and an experimental group in which BMDCs were treated first with R848 and then treated with poly(I:C) after a period of time (poly(I:C)) after R848). As a control for asynchronous treatment, cells simultaneously treated with R848 and poly(I:C) were used, and the amount of IL-12 secreted in synchronous treatment was set as 100, and results were converted to relative values. In addition, for synchronous treatment groups, which are controls, controls treated with R848 or poly(I:C) alone, and a control simultaneously treated with R848 and poly(I:C) (R848+poly(I:C)) were used, and an experimental group treated with a nanoliposome including a cholesterol-resiquimod complex and poly(I:C) (t-TLR7/8a+poly(I:C)) was prepared. Then, after the completion of the treatment, the cells were cultured for 36 hours, thereby obtaining a cell culture. The cell culture was centrifuged at 1,500 rpm for 3 minutes to separate cells and a supernatant, and the amount of secreted IL-12 included in the supernatant was measured through ELISA. The result is shown in FIGS. 11.

As shown in FIGS. 11, in the experimental group treated with R848 first and then poly(I:C), compared with the control co-treated with R848 and poly(I:C), it was confirmed that the amount of IL-12 secretion was reduced. That is, it was confirmed that there is no synergistic effect between the toll-like receptor 3 agonist and the toll-like receptor 7 or 8 agonist. However, in contrast, in the experimental group treated with poly(I:C) first and R848 two to four hours later, it was confirmed that IL-12 secretion was increased approximately 130% as compared with the control co-treated with R848 and poly(I:C). From the result, it was confirmed that when a first adjuvant is treated and then a second adjuvant acts two to four hours after the action of the first adjuvant, an immune activation action is maximized.

In addition, in the synchronous treatment experiment, in the experimental group co-treated with a nanoliposome and poly(I:C), it was confirmed that the highest level of IL-2 expression is shown. This means that the poly(I:C) first acts, and then the nanoliposome including a cholesterol-toll-like receptor 7 or 8 agonist complex moves into cells, where cholesterol is separated through GILT, and thus inactive resiquimod is converted to an active state and exhibits activity, which is the same result as the experimental group treated with both at different times.

### 14.2. Confirmation of synergistic effect by combination of toll-like receptor 4 agonist and nanoliposome including kinetically acting cholesterol-toll-like receptor 7 or 8 agonist complex

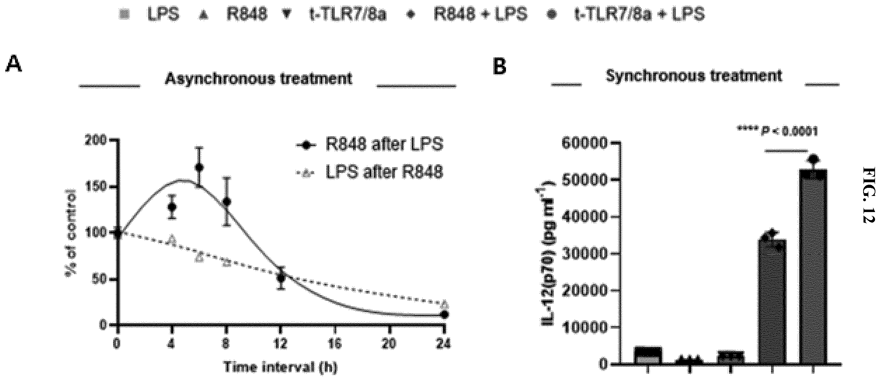

To confirm the synergistic effect of administration of a combination of adjuvants, experiments were conducted in the same manner as in Example 14.1 using the toll-like receptor 4 agonist LPS, the toll-like receptor 7 or 8 agonist resiquimod, and a nanoliposome including a cholesterol-toll-like receptor 7 or 8 agonist complex. The result is shown in FIGS. 12.

As shown in FIGS. 12, it was confirmed that, in the experimental group treated with LPS and then R848 approximately four hours later, the highest amount of IL-12 secretion is shown, and in the synchronous experiment, in the experimental group treated with LPS and a nanoliposome including kinetically acting cholesterol-toll-like receptor 7 or 8 agonist complex at the same time, the highest amount of IL-12 secretion is shown.

From the results, it can be seen that an order and time difference are important factors in the synergistic effect of adjuvants. That is, it was confirmed that, when immune activation is induced using a combination of adjuvants, to increase its effect, kinetic control that can adjust the activation time of each adjuvant is important.

### Example 15: Confirmation of immune activation efficacy by adjuvant ensemble

### 15.1. Confirmation of immune activation efficacy by combination of nanoliposome including kinetically acting cholesterol-toll-like receptor 7 or 8 agonist complex, and toll-like receptor 3 agonist or toll-like receptor 4 agonist

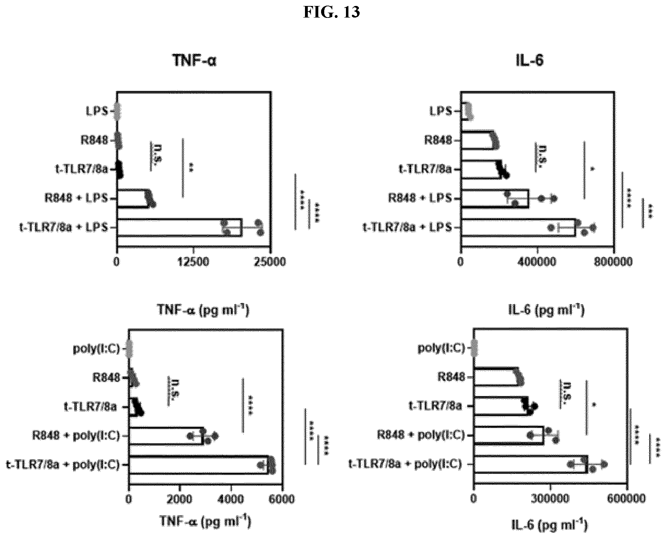

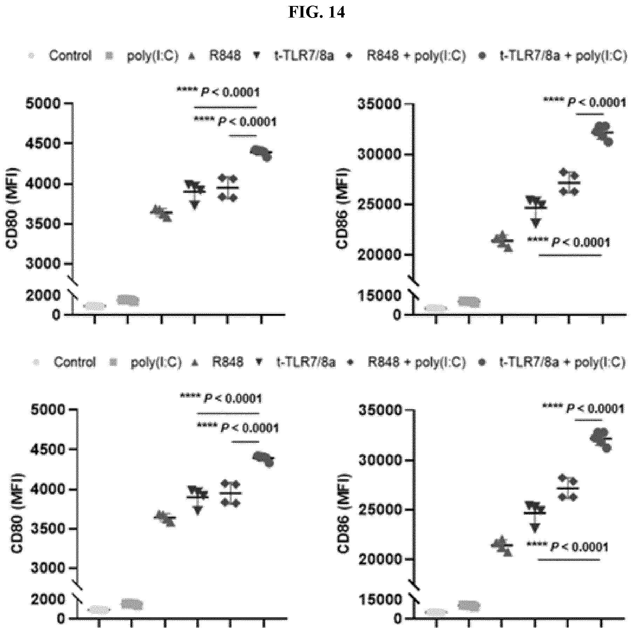

To confirm the immune activation efficacy by co-administration of a first adjuvant and a kinetically acting second adjuvant, as the first adjuvant, poly(I:C) or LPS was used, and as the second adjuvant, a nanoliposome including a cholesterol-toll-like receptor 7 or 8 agonist complex was used for experiments. Controls in which BMDCs are treated with LPS, poly(I:C), R848, or a nanoliposome including a cholesterol-toll-like receptor 7 or 8 agonist complex, an experimental group in which BMDCs are co-treated with LPS and R848, an experimental group in which BMDCs are co-treated with poly(I:C) and R848, an experimental group in which BMDCs are co-treated with LPS and a nanoliposome including a cholesterol-toll-like receptor 7 or 8 agonist complex, and an experimental group in which BMDCs are co-treated with poly(I:C) and a nanoliposome including a cholesterol-toll-like receptor 7 or 8 agonist complex were prepared, and cultured for 36 hours, thereby obtaining a cell culture. The obtained cell culture was centrifuged at 1,500 rpm for 3 minutes to separate cells and a supernatant, amounts of tumor necrosis factor (TNF-α) and IL-6 secretion included in the supernatant were measured, the cells were labeled using fluorescence-tagged antibodies, and then the maturation degree of the cells was confirmed by checking the expression of co-stimulatory molecules in dendritic cells using a BD Canto II flow cytometer. The result is shown in FIGS. 13 and 14.

As shown in FIG. 13, it was confirmed that, compared with the controls treated with a single adjuvant, in the experimental groups co-treated with adjuvants, the amounts of TNF-α and IL-6 secretion were increased, but in the experimental group co-treated with a nanoliposome including a kinetically acting cholesterol-toll-like receptor 7 or 8 agonist complex and an adjuvant, remarkably high amounts of TNF-α and IL-6 secretion are shown.

In addition, as shown in FIG. 14, it was confirmed that, in the experimental group co-treated with a nanoliposome including a kinetically acting cholesterol-toll-like receptor 7 or 8 agonist complex and an adjuvant, the highest expression levels of co-stimulatory molecules are shown.

From the results, it was confirmed that, by co-administration of a kinetically controllable adjuvant which is maintained inactive immediately after administration and induces an immune response delayed approximately 2 to 4 hours later and a general adjuvant, compared with the case of simply treating each adjuvant in combination, a remarkably increased immune activation effect can be exhibited.

### 15.2. Confirmation of effect of inducing continuous immune response by combination of nanoliposome including kinetically acting cholesterol-toll-like receptor 7 or 8 agonist complex, and toll-like receptor 3 agonist or toll-like receptor 4 agonist

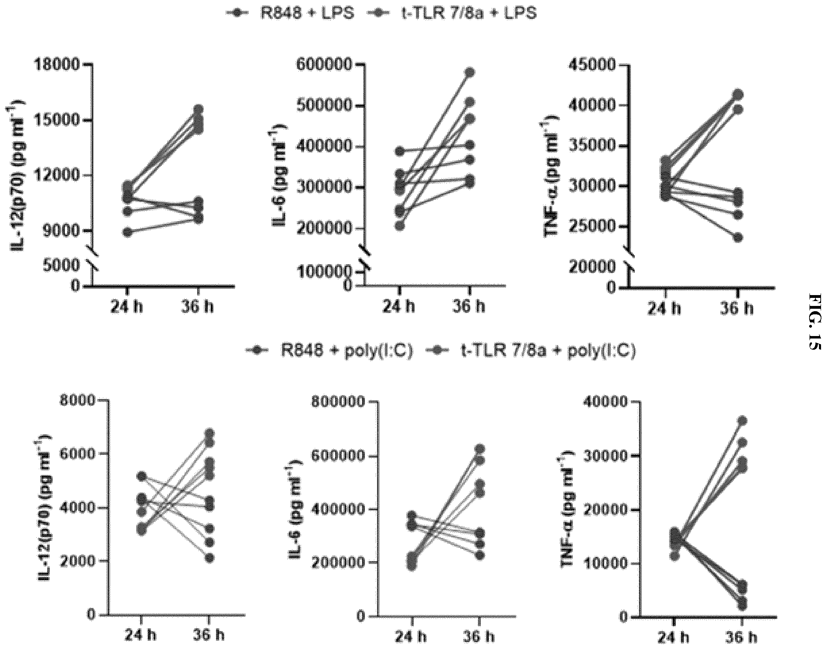

To confirm an immune activation efficacy by co-administration of a first adjuvant and a kinetically acting second adjuvant, experiments were conducted using poly(I:C) or LPS as a first adjuvant and a nanoliposome including a cholesterol-toll-like receptor 7 or 8 agonist complex as a second adjuvant. In further detail, BMDCs were treated with LPS and R848, poly(I:C) and R848, LPS and a nanoliposome including a cholesterol-toll-like receptor 7 or 8 agonist complex, or poly(I:C) and a nanoliposome including a cholesterol-toll-like receptor 7 or 8 agonist complex, and 24 and 36 hours later, a cell culture was obtained. The obtained cell culture was centrifuged at 1,500 rpm for 3 minutes to separate cells and a supernatant, the amounts of secreted IL-12, IL-6, and TNF-α included in the supernatant were measured by ELISA. The result is shown in FIG. 15.

As shown in FIG. 15, it was confirmed that, in the experimental group co-treated with poly(I:C) or LPS, and R848, the amounts of cytokines secreted at 24 hours and 36 hours were similar or reduced in all samples, but for the experimental group co-treated with poly(I:C) or LPS, and a nanoliposome including a cholesterol-toll-like receptor 7 or 8 agonist, the amounts of cytokines secreted after 36 hours are higher than after 24 hours in all samples. From the result, it was confirmed that a continuous immune response can be induced by co-administration of a kinetically acting second adjuvant and a first adjuvant.

### Example 16: Preparation of adjuvant ensemble system

### 16.1. Preparation of adjuvant mixture consisting of toll-like receptor 3 agonist and nanoliposome including kinetically acting cholesterol-toll-like receptor 7 or 8 agonist complex

A nanoliposome including the complex in which cholesterol and resiquimod are conjugated by a disulfide bond, prepared in the same manner as in Example 11.1 and poly(I:C) were mixed at a mass ratio of 4 : 1, and then mixed using a vortex mixer (3 sec) to prepare an adjuvant mixture in the stable form of particles. The mass of nanoliposome in the specification was calculated by conversion to the mass of the toll-like receptor 7 or 8 agonist included in the nanoliposome.

### 16.2. Confirmation of degree of stabilization of adjuvant mixture

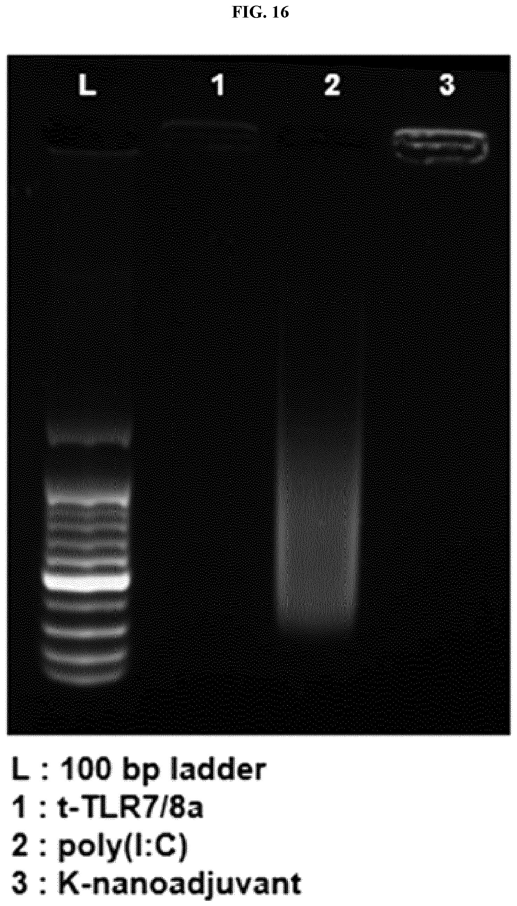

To confirm whether the adjuvant mixture prepared in the same manner as in Example 16.1 formed a stable mixture by electrical attraction, whether poly(1:C) not forming a mixture remains in the surroundings was confirmed using electrophoresis mobility shift assay (EMSA). In further detail, agarose (1 g) was added to a TAE buffer and dissolved by heating, poured into a mold, solidified for approximately 30 minutes, thereby preparing an agarose gel. Then, a size marker (100 bp), a nanoliposome including a cholesterol-resiquimod complex, poly(I:C), and the mixture (K-nanoadjuvant) were sequentially treated, and then electrophoresis was performed for 1 hour. The result is shown in FIG. 16.

As shown in FIG. 16, it was confirmed that all of the poly(I:C) is bound in a kinetically acting adjuvant mixture consisting of poly(I:C) and a nanoliposome including a cholesterol-resiquimod complex to form a stable mixture.

### 16.3. Confirmation of intracellular stability of adjuvant mixture

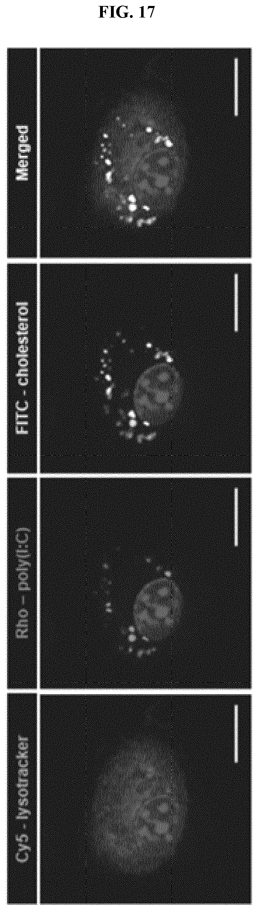

To confirm whether the adjuvant mixture is maintained in a stable mixed state even in cells, using R848, FITC-conjugated cholesterol (chol-FITC), and rhodamine-conjugated poly(I:C) (rhodamine-poly(I:C)), an adjuvant mixture was prepared in the same manner as in Example 16.1. Then, BMDCs (4×10⁴ cells/well) were seeded in an ibidi µ-slide-8-well microscopy chamber, and treated with the adjuvant mixture. Subsequently, after culture at 37 °C for 4 hours and washing with PBS, an endolysosome was stained with LysoTracker DeepRed, and a nucleus was stained with Hoechst 33342. Subsequently, DeltaVision PD was used to obtain a cell image. The result is shown in FIG. 17.

As shown in FIG. 17, it was confirmed that a stable mixture is maintained even in cells by detecting the fluorescence of the nanoliposome including chol-FITC and the fluorescence of rhodamine-poly(I:C) at the same location.

### Example 17: Confirmation of efficacy of adjuvant mixture

### 17.1. Confirmation of continuous immune response-inducing efficacy of adjuvant mixture in tumor-draining lymph node

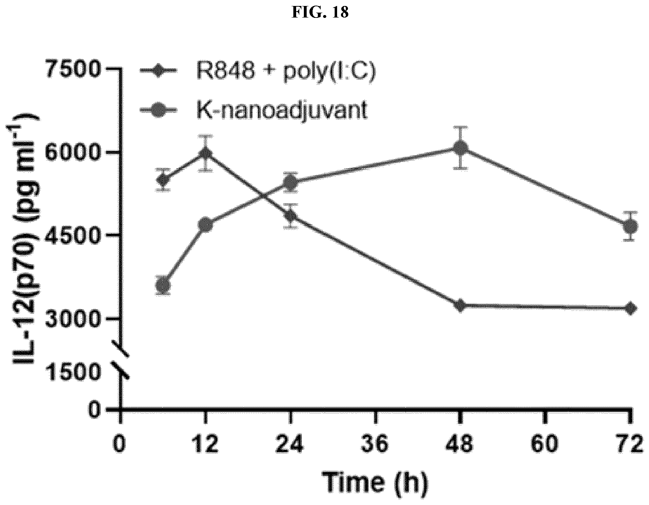

To confirm the efficacy of inducing an immune response of the adjuvant mixture prepared in the same manner as in Example 16.1 in a tumor-draining lymph node, R848 (25 µg), poly(I:C) (6.25 µg), or an adjuvant mixture including the same amounts of R848 and poly(I:C) was mixed with SIINFEKL antigen and then subcutaneously injected into C57BL/6 mice via single inoculation. Subsequently, 6, 12, 24, 48, and 72 hours after inoculation, a lymph node was extracted, and the extracted lymph node was suspended in a cell lysis buffer (CellLytic MT cell lysis), and mechanically disrupted. The cell-disrupted solution was centrifuged at 4 °C and 10,000xg for 10 minutes, thereby obtaining an impurity-removed supernatant. Then, the amount of IL-12p70 included in the obtained supernatant was confirmed by ELISA. The result is shown in FIG. 18.

As shown in FIG. 18, it was confirmed that, in the control co-treated with R848 and poly(I:C), IL-12p70 secretion in the lymph node is reduced after 12 hours, and in the experimental group treated with the adjuvant mixture, IL-12p70 secretion is continuously increased until 48 hours. From the result, it was confirmed that, since the kinetically acting adjuvant mixture has different action times for the first adjuvant and the second adjuvant, an immune response is continuously induced in the body.

### 17.2. Confirmation of antitumor efficacy of adjuvant mixture

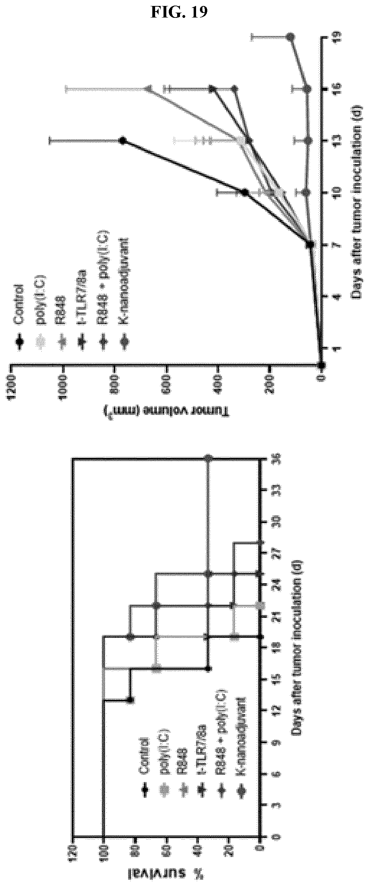

To confirm the antitumor efficacy of the adjuvant mixture prepared in the same manner as in Example 16.1, first, 5×10⁵ B16OVA melanoma cells were subcutaneously inoculated into the right flank of a mouse to manufacture a cancer animal model. Subsequently, R848 (25 µg), poly(I:C) (6.25 µg), or an adjuvant mixture including the same amounts of R848 and poly(I:C) was mixed with SIINFEKL antigen and then injected into a cancer animal model once every 3 days for single inoculation. Then, tumor size and viability were continuously checked. A tumor volume was calculated by "major axis diameter X minor axis diameter²/2." The result is shown in FIG. 19.

As shown in FIG. 19, it was confirmed that 2 out of 5 mice administered the adjuvant mixture survived until 36 days, and an increase in tumor volume was effectively inhibited. From the results, it was confirmed that the anticancer effect was remarkably increased using a kinetically acting adjuvant mixture.

### 17.3. Confirmation of immune response-inducing efficacy of adjuvant mixture in tumor-draining lymph node and tumor microenvironment

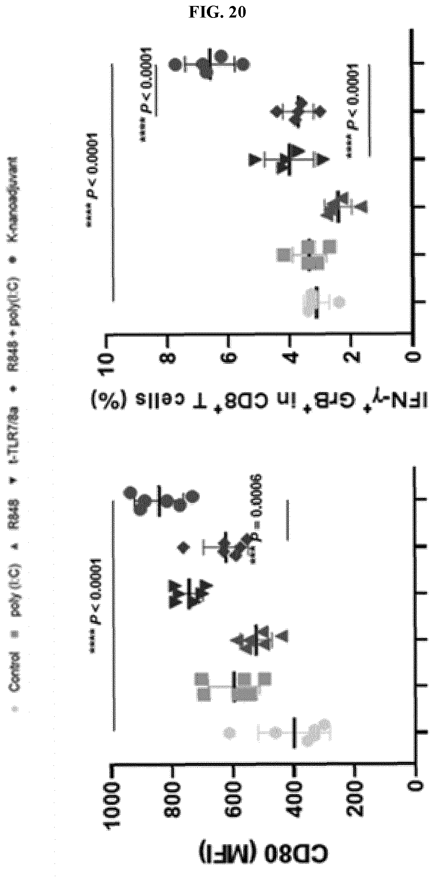

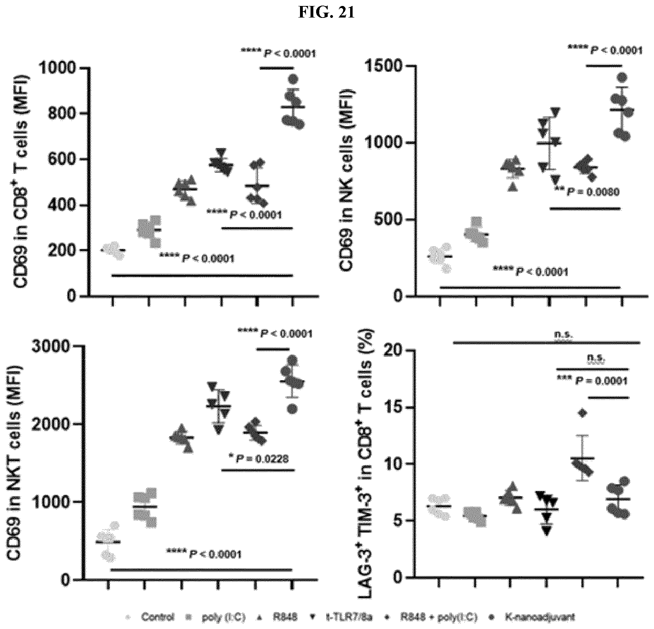

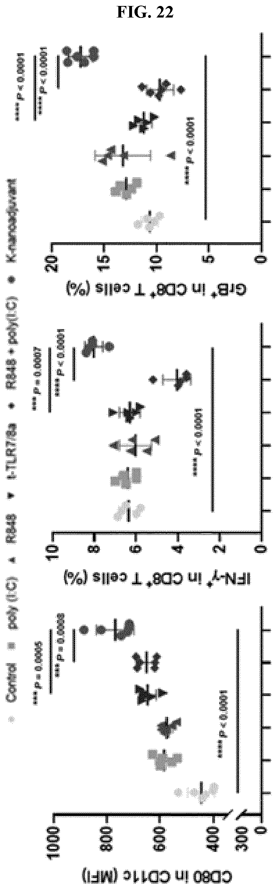

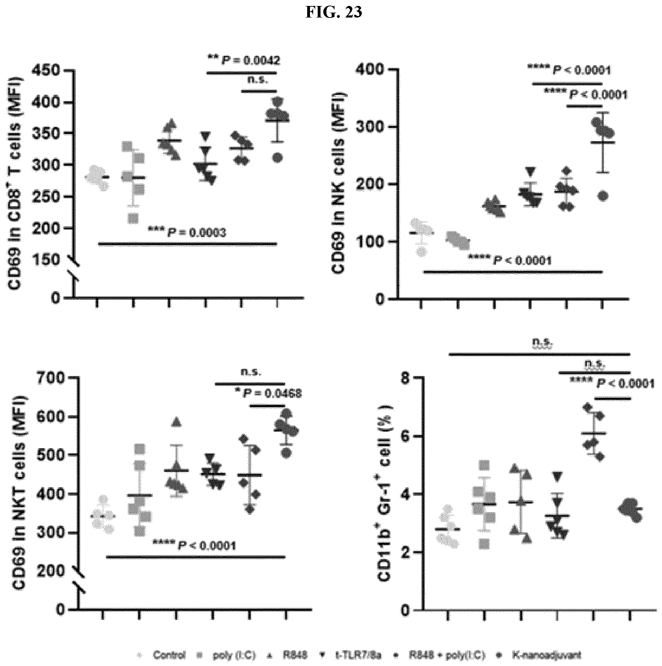

To confirm the immune response-inducing efficacy of the adjuvant mixture prepared in the same manner as in Example 16.1 in a tumor-draining lymph node (TDLN) and tumor microenvironment (TME), first, 5×10⁵ B16OVA melanoma cells were subcutaneously inoculated into the right flank of a mouse to manufacture a cancer animal model. Subsequently, in the cancer animal model, R848 (25 µg), poly(I:C) (6.25 µg), or an adjuvant mixture including the same amounts of R848 and poly(I:C) were mixed with SIINFEKL antigen and then subcutaneously injected three times at intervals of three days for single inoculation. Then, three days after the last injection, tumor tissue and a tumor-draining lymph node were extracted. Then, to analyze immune cells recruited to the tumor tissue and the lymph node, the tumor tissue and the lymph node were mechanically disrupted, resuspended in a collagenase D (1 mg/mL)-supplemented medium, and incubated in a shaking incubator at 37 °C for 40 minutes. Afterward, the resulting sample was filtered using a 70-µm cell strainer and washed twice with PBS, thereby obtaining single cells. The obtained single cells were stained with various fluorescence-tagged antibodies, and analyzed by a BD Canto II flow cytometer. The result is shown in FIGS. 20 to 23.

As shown in FIGS. 20 to 23, it was confirmed that the kinetically acting adjuvant mixture generates mature dendritic cells in the tumor-draining lymph node. In further detail, in the tumor tissue and tumor-draining lymph node of a mouse administered the adjuvant mixture, compared with the experimental group simultaneously administered R848 and poly(I:C), it was confirmed that multifunctional IFN-γ⁺ Granzyme-B⁺ and CD69 were induced in CD8⁺ T cells at considerably high frequency. In addition, it was confirmed that, in CD8⁺ T cells of the experimental group co-treated with R848 and poly(I:C), the expression levels of PD-1, TIM-3, and LAG-3 indicating the depleted state of the T cells increase, but in the experimental group treated with the adjuvant mixture, the expression levels were similar to that of the control (treated with PBS). In addition, it was confirmed that CD69 expression was induced in CD4⁺ T cells, CD8⁺ T cells, natural killer cells (NK cells), and natural killer T cells, and the generation of myeloid-derived suppressor cells (MDSCs), representing immunosuppressive cells, was inhibited. From the results, it was confirmed that the adjuvant mixture of the present invention inhibits the depleted state of T cells *in vivo*, improves an immune activation effect, and prolongs the duration of an immune response, thereby exhibiting a remarkably high immune modulation effect, compared with simple co-administration of adjuvants.

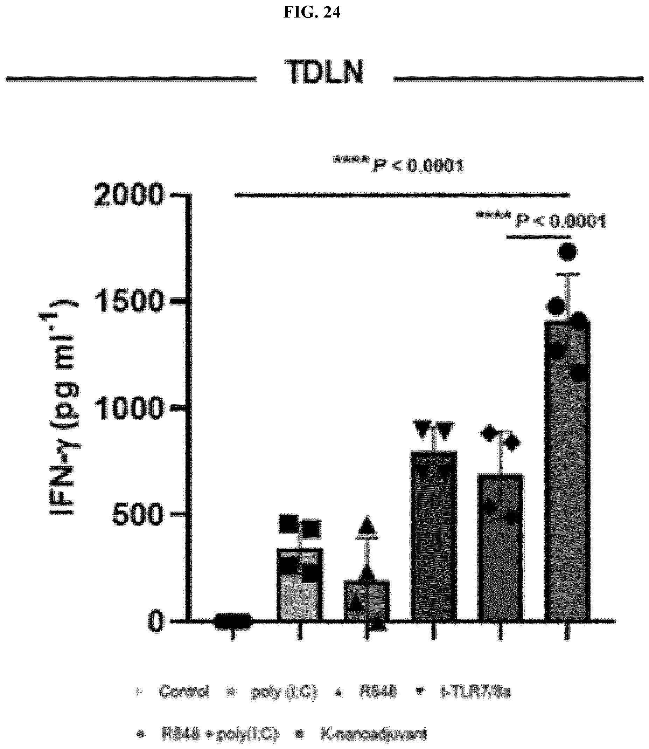

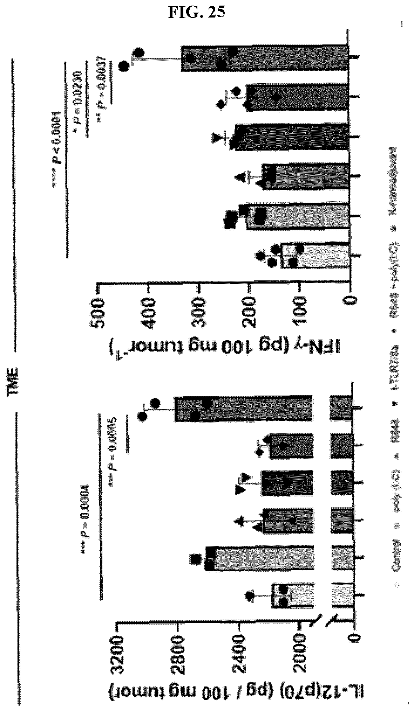

In addition, to confirm cytokines secreted from tumor tissue and lymph node tissue, the obtained tumor tissue was suspended in a cell lysis buffer (CellLytic MT cell lysis), and mechanically disrupted. In addition, the resulting cells were centrifuged at 4 °C and 10,000xg for 10 minutes, thereby obtaining a supernatant. In addition, the lymph node was mechanically disrupted, and resuspended in a collagenase D (1 mg/mL)-supplemented medium. In addition, the resuspended solution was incubated in a shaking incubator at 37 °C for 40 minutes, and filtered using a 70-µm cell strainer. In addition, the resultant was washed twice with PBS, centrifuged at 1,500 rpm for 3 minutes to obtain single cells, the cells were cultured at 37 °C for 24 hours, and the cell culture was centrifuged to obtain a supernatant. In addition, the amounts of secreted IL-12p70 and IFN-γ included in the obtained supernatant were confirmed by ELISA. The result is shown in FIGS. 24 and 25.

As shown in FIGS. 24 and 25, it was confirmed that the adjuvant mixture effectively induces the secretion of both IL-12p70 and IFN-γ in the tumor tissue and tumor-draining lymph node.

From the results, it was confirmed that the kinetically acting adjuvant mixture improves the proliferative capability of various immune cells, increases cytokine productivity, and increases lytic granules without inducing CD8⁺ T cell depletion, thereby improving the effector function of the CD8⁺ T cells.

### Example 18: Confirmation of efficacy of adjuvant mixture using IL-12 neutralizing antibodies

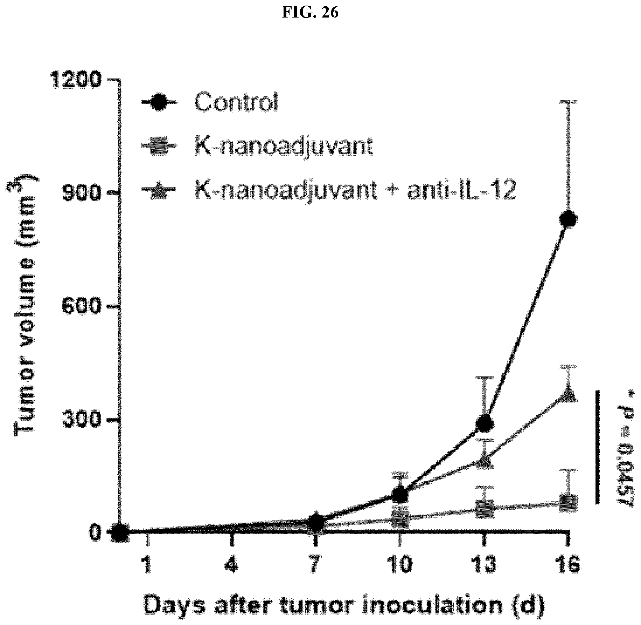

To confirm whether the proliferation and immune activation of immune cells improved by the adjuvant mixture are mediated by continuous production of IL-12p70, first, 5×10⁵ B16OVA melanoma cells were subcutaneously inoculated into the right flank of a mouse to manufacture a cancer animal model. Subsequently, before inoculation of the cancer animal model with the adjuvant mixture, anti-mouse IL-12p75 (300 µg) was administered intraperitoneally five times at intervals of three days. Then, three days after the fifth injection, the adjuvant mixture was mixed with SIINFEKL antigen, and subcutaneously injected three times at intervals of three days via single inoculation. Then, tumor size and viability were continuously checked. The result is shown in FIG 26.

As shown in FIG. 26, it was confirmed that the antitumor effect of the adjuvant mixture is reduced in the anti-IL-12-treated experimental group. From the result, it was confirmed that IL-12 plays an important role between the stimulation of innate immunity and an adaptive immune response of a kinetically acting adjuvant mixture consisting of a toll-like receptor 3 agonist and a nanoliposome including a cholesterol-toll-like receptor 7 or 8 agonist complex.

### Example 19: Confirmation of antitumor immune response-inducing effect via local inoculation of adjuvant mixture

### 19.1. Confirmation of antitumor efficacy on metastatic cancer via local inoculation of adjuvant mixture

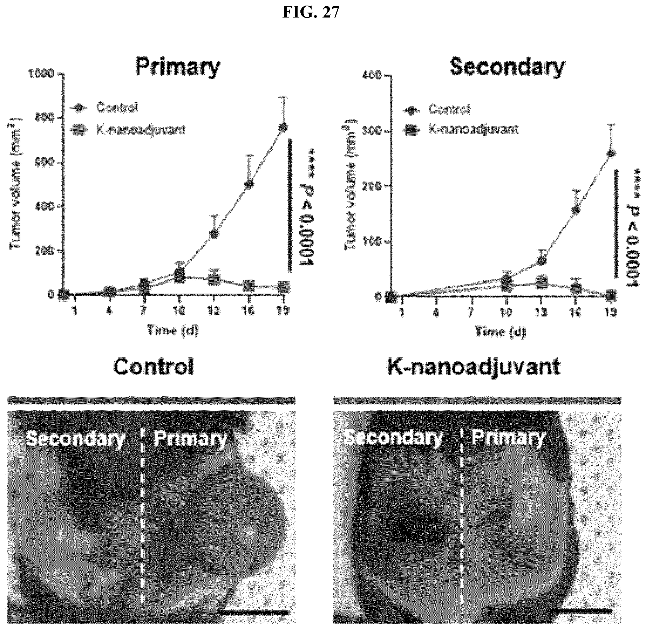

To confirm the antitumor efficacy against metastatic cancer via local inoculation with the adjuvant mixture, 5×10⁵ TC-1 cells were subcutaneously inoculated into the right flank of a mouse to manufacture a cancer animal model. Then, after 4 days, 2.5×10⁵ TC-1 cells were subcutaneously inoculated into the left flank of the mouse. Subsequently, the adjuvant mixture was mixed with SIINFEKL antigen and then subcutaneously injected into the cancer animal model four times at intervals of three days via single inoculation. Then, three days after the last inoculation, tumor tissue and a tumor-draining lymph node were extracted. Tumor size and viability were continuously checked. The result is shown in FIG. 27.

As shown in FIG. 27, it was confirmed that, in the experimental group inoculated with the adjuvant mixture, the growth of a primary tumor as well as a secondary tumor is effectively inhibited.

### 19.2. Confirmation of lung metastasis inhibitory effect through local inoculation with adjuvant mixture

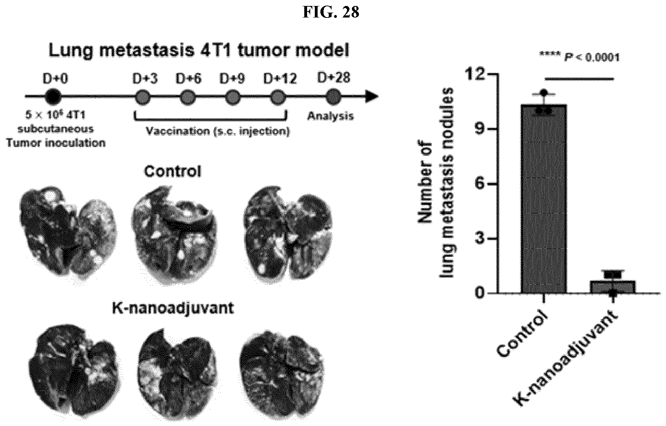

To confirm whether local inoculation with an adjuvant mixture inhibits cancer metastasis, 5×10⁵ 4T1 cells were subcutaneously inoculated into the right flank of a mouse to manufacture a cancer animal model. Subsequently, after 5 days, a tumor lysate (10 µg) was mixed with the adjuvant mixture and subcutaneously injected four times at intervals of three days for single inoculation. Then, after 30 days, the mouse was euthanized and then a mixed solution of India pink (47 mL) and PBS (3 mL) was injected to stain lungs. The stained lung was extracted, washed with PBS, and fixed in a fixation solution (70% ethane (40 mL), 4% formaldehyde (1 mL) and acetic acid (0.5 mL)), and then lung metastatic nodules were counted by visual observation. The result is shown in FIG. 28.

As shown in FIG. 28, multiple lung metastatic tumor nodules were observed in the control group, but it was confirmed that lung metastasis was not observed in the experimental group administered the adjuvant mixture.

### 19.3. Confirmation of antitumor efficacy against orthotopic tumor via local inoculation with adjuvant mixture

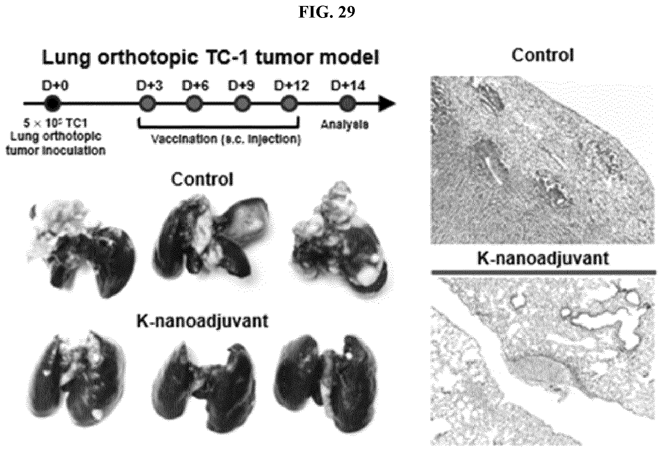

To confirm the antitumor efficacy against an orthotopic tumor via local inoculation with the adjuvant mixture, first, a C57BL/6 mouse was anesthetized with respiratory anesthesia. Subsequently, after incising the right side of the chest skin, 5×10⁵ TC-1 cells were inoculated into the right side of the lung lobe. Then, after three days, an adjuvant mixture was mixed with Long-E7 peptide, and subcutaneously injected four times at intervals of three days for single inoculation. Then, three days after the last inoculation, the mouse was euthanized, the lungs were extracted, and then the extracted lungs were sliced and stained with hematoxylin and eosin. The result is shown in FIG. 29.

As shown in FIG. 29, it was confirmed that tumor cells directly inoculated into the lung grew aggressively in the untreated control, whereas tumor growth was completely inhibited in the adjuvant mixture-administered experimental group.

From the results, it was confirmed that a systemic antitumor immune response was induced also by the local administration of a kinetically acting adjuvant mixture, effectively exhibiting an antitumor effect.

### Example 20: Confirmation of antitumor efficacy by combination of adjuvant mixture and immune checkpoint inhibitor therapy or chemotherapy

To confirm whether a therapeutic effect on various anticancer treatments through the immune activation action of the adjuvant mixture, experiments were conducted.

### 20.1. Confirmation of antitumor efficacy by combination of adjuvant mixture and immune checkpoint inhibitor therapy

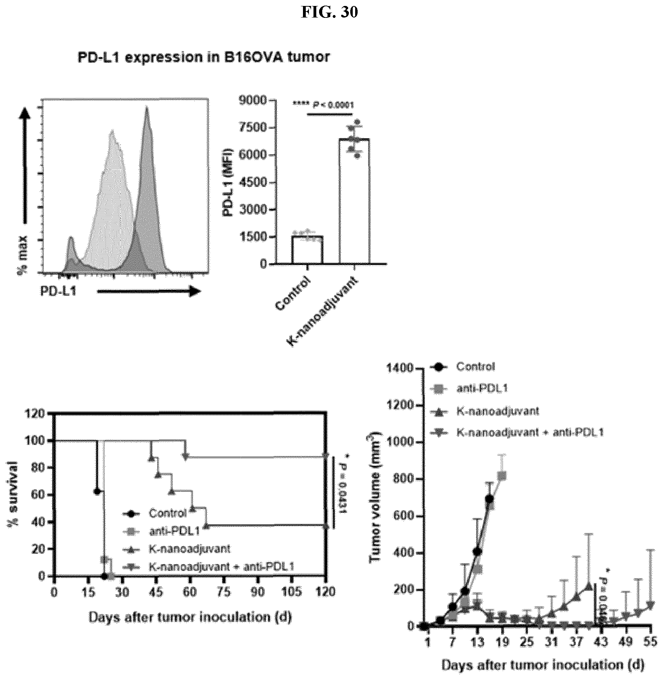

To confirm the antitumor efficacy by the combination of an adjuvant mixture and immune checkpoint inhibitor therapy (ICBT therapy), first, 5×10⁵ TC-1 cells were subcutaneously inoculated into the right flank of a mouse to manufacture a cancer animal model. Subsequently, after 4 days, the adjuvant mixture and Long-E7 peptide were mixed and injected subcutaneously 6 times at intervals of three days for single inoculation. Then, anti-PD-L1 was administered intraperitoneally 8 times at intervals of two days. Three days after the final administration, the degree of PD-L1 expression in tumor tissue was confirmed, and tumor size and viability were continuously checked. The result is shown in FIG. 30.

As shown in FIG. 30, in the case of the combination of the adjuvant mixture and immune checkpoint inhibitor therapy, it was confirmed that PD-L1 expression was remarkably increased, and cancer growth was also effectively inhibited. In addition, in the case of the combination of the adjuvant mixture and immune checkpoint inhibitor therapy, it was confirmed that the mouse survived for more than 120 days. From the result, it was confirmed that the adjuvant mixture induces an increase in activity of cytotoxic lymphocytes and an increase in IFN-γ secretion, thereby increasing PD-L1 expression, and modulates an immune response in a tumor microenvironment, thereby further increasing the anticancer effect by immune checkpoint inhibitor therapy.

### 20.2. Confirmation of antitumor efficacy by combination of adjuvant mixture and chemotherapy

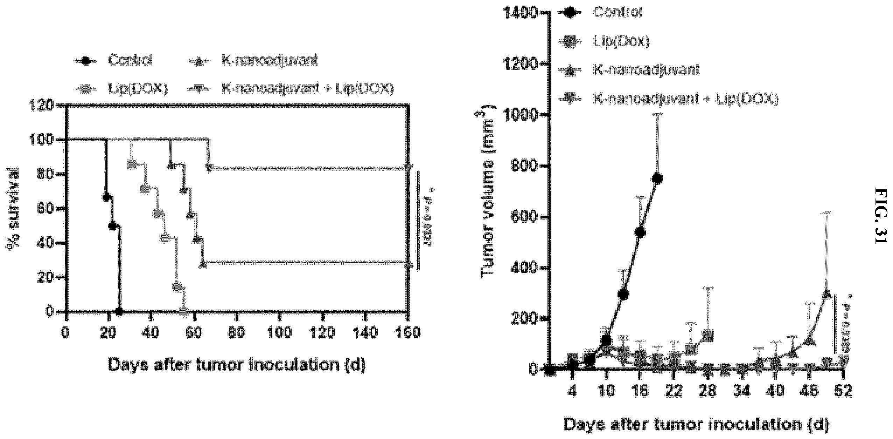

To confirm the anti-tumor efficacy of the combination of an adjuvant mixture and chemotherapy, first, 5×10⁵ TC-1 cells were subcutaneously inoculated into the right flank of a mouse to manufacture a cancer animal model. Subsequently, after 4 days, the adjuvant mixture and Long-E7 peptide were mixed and injected subcutaneously five times at intervals of three days for single inoculation. Then, a doxorubicin liposome formulation (80 µg) was intravenously injected twice at intervals of six days. Then, tumor size and viability were continuously checked. The result is shown in FIG. 31.

As shown in FIG. 31, in the case of the combination of the adjuvant mixture and chemotherapy, it was confirmed that cancer growth is effectively inhibited. In addition, in the combination of the adjuvant mixture and chemotherapy, it was confirmed that the mouse survived for more than 160 days. From the results, it was confirmed that cancer can be effectively treated by combined therapy of the adjuvant mixture and chemotherapy.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not limited in any aspect.

### [Industrial Applicability]

When two or more types of immune activating materials were administered at the same time, a kinetically acting adjuvant ensemble composition of the present invention may provide a kinetic ensemble acting at different times, thereby eliciting the maximum synergistic effect of immunological responses, and may be modulated to sequentially secrete various functional drugs easily included in a drug delivery system, and thus not only can it be applied to various diseases that can be treated using an adjuvant, but also a therapeutic effect can be remarkably increased.

## Claims

1. A kinetically acting adjuvant ensemble composition, comprising two or more types of adjuvants,
in which a first adjuvant binds to an immune cell receptor to induce a first immune response, and
a second adjuvant is a complex in which a cleavable linker binds to its active site, sequentially binds to an immune cell receptor to induce a second immune response.

2. The adjuvant ensemble composition of claim 1, wherein the kinetic action appears in such a manner that when the cleavable linker is bound to the active site of the second adjuvant to maintain an inactive state, and then the cleavable linker blocking the active site is cleaved within 2 to 12 hours, the activity of the immune activating material is temporally delayed.

3. The adjuvant ensemble composition of claim 1, wherein the cleavable linker comprises any one or more bonds selected from the group consisting of disulfide, carbamate, hydrazine, ester, peptide, azide, amide, hydrazone, thioether, phosphodiester, thioketal, and a combination thereof.

4. The adjuvant ensemble composition of claim 1, wherein the cleavable linker further comprises ethylene oxide or ethylene glycol at one or both ends thereof.

5. The adjuvant ensemble composition of claim 1, wherein the cleavable linker is cleaved at the chemical bond of a binding site due to any one or more factors selected from the group consisting of an enzyme, pH, redox potential, a temperature, ultrasonic wave, magnetic force, and a light source.

6. The adjuvant ensemble composition of claim 1, wherein any one or more materials selected from the group consisting of cholesterol, a lipid, a protein, an amino acid, a peptide and an oligonucleotide is bound to an end of the cleavable linker.

7. The adjuvant ensemble composition of claim 1, wherein the second adjuvant is loaded in any one or more drug delivery systems selected from the group consisting of a nanoliposome, a nanoemulsion, a nanomicelle, a hydrogel, a scaffold, a solid nanoparticle and a polymeric nanoparticle.

8. The adjuvant ensemble composition of claim 7, wherein the drug delivery system further comprises a first adjuvant.

9. The adjuvant ensemble composition of claim 7, wherein the drug delivery system further comprises a ligand that is able to react with a receptor present on the surface of immune cells or in an endosome or cytosol.

10. The adjuvant ensemble composition of claim 7, wherein the drug delivery system further comprises any one or more immune activating materials selected from the group consisting of a toll-like receptor agonist, saponin, an antiviral peptide, an inflammasome inducer, a NOD ligand, a cytosolic DNA sensor (CDS ligand), a stimulator of interferon genes (STING) ligand, an outer wall component of a pathogen, alum, a lipid, a combination thereof, and a derivative thereof.

11. The adjuvant ensemble composition of claim 1, wherein the second adjuvant is a toll-like receptor agonist.

12. The adjuvant ensemble composition of claim 1, wherein the first adjuvant is any one or more immune activating materials selected from the group consisting of a toll-like receptor agonist, saponin, an antiviral peptide, an inflammasome inducer, a NOD ligand, a cytosolic DNA sensor (CDS ligand), a stimulator of interferon genes (STING) ligand, an outer wall component of a pathogen, an alum, a lipid, a combination thereof, and a derivative thereof.

13. The adjuvant ensemble composition of claim 1, wherein the immune cells are any one or more selected from the group consisting of antigen-presenting cells (dendritic cells or macrophages), natural killer (NK) cells, T cells, B cells, regulatory T cells, myeloid derived suppressor cells (MDSCs), and M2 macrophages.

14. A pharmaceutical composition for preventing or treating an infectious disease, a cancer, a metabolic syndrome, an autoimmune disease, or a rare disease, comprising the adjuvant ensemble composition of claim 1 as an active ingredient.

15. The pharmaceutical composition of claim 14, wherein the pharmaceutical composition further comprises an antigen, a chemotherapeutic agent, or an immune checkpoint inhibitor.

16. The pharmaceutical composition of claim 15, wherein the antigen is one or more selected from the group consisting of a protein, a recombinant protein, a glycoprotein, a gene, a peptide, a polysaccharide, a lipopolysaccharide, a polynucleotide, a cell, a cell lysate, a bacterium, and a virus.

17. The pharmaceutical composition of claim 14, wherein the pharmaceutical composition inhibits cancer growth, metastasis, recurrence, or resistance to anticancer therapy.

18. A method of preventing or treating an infectious disease, cancer, metabolic syndrome, an autoimmune disease, or a rare disease, comprising:
administering a composition comprising the adjuvant ensemble composition of claim 1 as an active ingredient to a subject.

19. A composition for use in preventing or treating an infectious disease, cancer, metabolic syndrome, an autoimmune disease, or a rare disease,
wherein the composition comprises the adjuvant ensemble composition of claim 1 as an active ingredient.

20. A use of the adjuvant ensemble composition of claim 1 for the manufacture of medicament for preventing or treating of an infectious disease, cancer, metabolic syndrome, an autoimmune disease, or a rare disease.
